# EUROPEAN PATENT APPLICATION

(11) **EP 4 806 458 A1**
(43) Date of publication of application: **16.09.2026**
(21) Application number: 24888068.4
(22) Date of filing: 07.11.2024
(51) Int. Cl.: A61K 38/28, A61P 3/10, C07K 14/62

(54) **ACYLATED INSULIN FOR REDUCING BLOOD GLUCOSE**

(30) Priority: 07.11.2023 CN 202311472986; 02.06.2024 CN 202410702372; 08.08.2024 CN 202411087070
(71) Applicant: Gan & Lee Pharmaceuticals Co., Ltd., Beijing 101109 (CN)
(72) Inventor: GAN, Zhongru, Beijing 101109 (CN); CHEN, Wei, Beijing 101109 (CN); ZHAO, Jing, Beijing 101109 (CN); HAO, Chunyue, Beijing 101109 (CN)
(74) Representative: Kraus & Lederer PartGmbB
(86) International application number: PCT/CN2024/130670
(87) International publication number: WO 2025/098457

(57) **Abstract**

The present invention relates to acylated insulin for reducing blood glucose. Provided is a method for treating or preventing metabolic syndrome and type II diabetes in a subject in need thereof by administering a compound of formula (I) or a pharmaceutically acceptable salt thereof. Further provided is a kit comprising an instruction for treatment according to the method.

## Description

This application claims priority to Chinese Patent Application No. 202311472986.8 filed on November 7, 2023, Chinese Patent Application No. 202410702372.2 filed on June 2, 2024, and Chinese Patent Application No. 202411087070.5 filed on August 8, 2024. The entire contents of the above three applications are incorporated herein by reference.

### TECHNICAL FIELD

The present invention relates to the field of biopharmaceuticals and relates to acylated insulin for the treatment of type 2 diabetes.

### BACKGROUND

Diabetes is a metabolic disorder characterized by hyperglycemia. Optimal glycemic control is the therapeutic goal for patients with type 2 diabetes. Despite the availability of several oral antidiabetic drugs and insulin, a significant proportion of type 2 diabetes patients fail to achieve the recommended glycemic control targets.

Insulin is used to treat diabetes and diseases associated with or caused by it, and is essential for maintaining normal metabolic regulation. However, natural insulin such as human insulin has a relatively short duration of action, requiring patients to administer frequent injections and causing numerous injection-related discomforts. Therefore, people have been committed to developing insulin derivatives or analogues with superior efficacy, longer duration of action, and reduced injection frequency to alleviate the inconvenience and discomfort associated with high-frequency insulin injections.

WO1995007931A1 discloses the commercially available long-acting insulin detemir, whose molecular structure is characterized by the removal of threonine at position 30 of the human insulin B chain and the attachment of a 14-carbon fatty acid to the lysine residue at position 29 of the B chain. WO2005012347A2 discloses another commercially available long-acting insulin degludec. Insulin degludec is a new type of ultra-long-acting insulin with a longer duration of action than insulin detemir. Its molecular structure is characterized by the removal of the threonine at position 30 of the human insulin B-chain and the attachment of a 16-carbon fatty diacid side chain to the lysine residue at position 29 of the B-chain via a glutamic acid molecule. CN101573133B and WO2009/010428 disclose PEG (PEGylated)-extended insulin, and compared to conventional unmodified insulin, PEG-extended insulin has a longer duration of action. WO2013086927A1 and WO2018/024186 disclose acylated derivatives of a long-acting human insulin analog.

However, to date, no basal insulin product that requires administration less frequently than once daily via subcutaneous injection has been approved for marketing.

Therefore, there remains a need for insulin derivatives or analogs that have better therapeutic efficacy or potency, longer duration of action, lower administration frequency, and superior physicochemical properties compared to currently available insulins (such as degludec) or known insulin derivatives.

### SUMMARY

The disclosure relates to a method for treating or preventing metabolic syndrome, which comprises administering a therapeutically effective amount of compound (I) to a subject in need thereof who has clinical symptoms of hyperglycemia; wherein the method reduces the occurrence of adverse events of hyperglycemia (MACE). The dosing regimen described herein provides benefits for the use of the compound of formula (I) in the treatment of type II diabetes, maximizes its hypoglycemic efficacy, and reduces or eliminates the adverse side effects caused by its hypoglycemic effect.

A first aspect of the present disclosure provides a method for treating or preventing metabolic syndrome, wherein the method comprises administering about 5-400U, preferably about 5-120U, of a compound of formula (I), or a pharmaceutically acceptable salt, amide, or ester thereof to a subject in need thereof; preferably, the method comprises administering a dose of about 5-400U, preferably about 5-120U, of the compound of formula (I), or the pharmaceutically acceptable salt, amide, or ester thereof, to the subject in need thereof at a frequency of once every three days, twice weekly, once every four days, once every five days, once weekly or less, wherein the dose for each administration is independently the same or different, 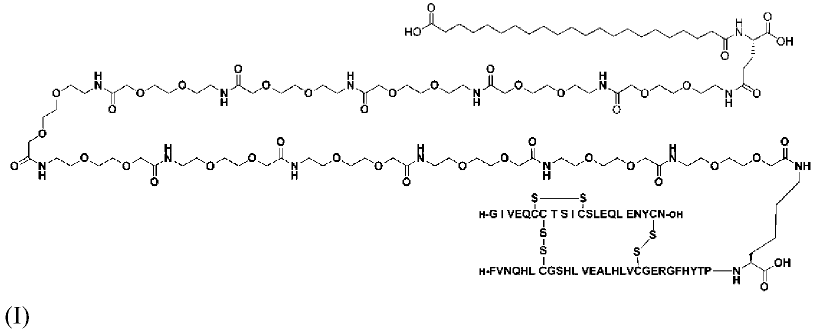
preferably, the metabolic syndrome is diabetes mellitus;
preferably, the diabetes mellitus is type II diabetes (type 2 diabetes);
preferably, the subject is a patient with type 2 diabetes who is insulin-naive and inadequately controlled on oral antidiabetic drugs;
preferably, the subject is a patient with type 2 diabetes who is inadequately controlled on a combination of oral antidiabetic drugs and basal insulin.

Further, wherein the method comprises administering the following dose of the compound of formula (I), or the pharmaceutically acceptable salt, amide, or ester thereof to the subject in need thereof: about 5-400U, about 5-350U, about 5-300U, preferably about 10-250U, preferably about 10-200U, preferably about 10-150U, preferably about 10-120U, preferably about 10-110U, preferably about 15-105U, preferably about 20-100U, preferably about 30-100U, preferably about 31-100U, preferably about 31-97U, preferably about 35-95U, preferably about 38-90U, preferably about 40-90U, preferably about 40-88U, preferably about 45-88U, preferably about 48-85U, preferably about 49-80U, preferably about 49-79U, preferably about 49-70U, preferably about 40-70U of the compound of formula (I), or the pharmaceutically acceptable salt, amide, or ester thereof; preferably, the method comprises administering the following dose of the compound of formula (I), or the pharmaceutically acceptable salt, amide, or ester thereof to the subject in need thereof at a frequency of once every three days, twice weekly, once every four days, once every five days, once weekly or less: about 5-400U, about 5-350U, about 5-300U, preferably about 10-250U, preferably about 10-200U, preferably about 10-150U, preferably about 10-120U, preferably about 10-110U, preferably about 15-105U, preferably about 20-100U, preferably about 30-100U, preferably about 31-100U, preferably about 31-97U, preferably about 35-95U, preferably about 38-90U, preferably about 40-90U, preferably about 40-88U, preferably about 45-88U, preferably about 48-85U, preferably about 49-80U, preferably about 49-79U, preferably about 49-70U, preferably about 40-70U of the compound of formula (I), or the pharmaceutically acceptable salt, amide, or ester thereof, wherein the dose for each administration is independently the same or different.

Further, wherein the method comprises administering to the subject in need thereof the compound of formula (I), or the pharmaceutically acceptable salt, amide, or ester thereof at a weekly dose of: about 5-300U, preferably about 10-250U, preferably about 10-200U, preferably about 10-150U, preferably about 10-120U, preferably about 10-110U, preferably about 15-105U, preferably about 20-100U, preferably about 30-100U, preferably about 31-100U, preferably about 31-97U, preferably about 35-95U, preferably about 38-90U, preferably about 40-90U, preferably about 40-88U, preferably about 45-88U, preferably about 48-85U, preferably about 49-80U, preferably about 49-79U, preferably about 49-70U, preferably about 40-70U, preferably each weekly dose is independently the same or different.

Further, wherein the method comprises administering to the subject in need thereof the compound of formula (I) or the pharmaceutically acceptable salt, amide, or ester thereof at the following weekly dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, preferably each weekly dose is independently the same or different.

Further, wherein the method comprises administering to the subject in need thereof the compound of formula (I) or the pharmaceutically acceptable salt, amide, or ester thereof at a frequency of once every three days, twice weekly, once every four days, once every five days, once weekly or less, preferably the dose for each administration is independently the same or different;
preferably, the method comprises administering to the subject in need thereof the following dose of the compound of formula (I) or the pharmaceutically acceptable salt, amide, or ester thereof at a frequency of once weekly: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, wherein each once-weekly dose is independently the same or different;
preferably, the method comprises administering to the subject in need thereof the compound of formula (I) or the pharmaceutically acceptable salt, amide, or ester thereof at a frequency of once weekly, at the following fixed dose based on body weight of the subject:
   about 6nmol-12nmol/kg, preferably about 6nmol/kg, about 7nmol/kg, about 8nmol/kg, about 9nmol/kg, about 10nmol/kg, about 11nmol/kg, or about 12nmol/kg; or
   about 1U/kg-6U/kg, preferably 1U/kg-3U/kg, preferably about 1U/kg-2U/kg, preferably about 1U/kg, about 1.3U/kg, about 1.5U/kg or about 2U/kg;
   preferably, for subjects who were previously administered once-daily or twice-daily basal insulin, when switching to the administration of the compound of formula (I) or the pharmaceutically acceptable salt, amide, or ester thereof, the dose of the compound of formula (I) or the pharmaceutically acceptable salt, amide, or ester thereof administered each time is about 2-20 times, preferably about 2-10 times, preferably about 2-7 times, the dose of previous basal insulin, preferably about 2 times, about 2.5 times, about 3 times, about 3.5 times, about 4 times, about 4.5 times, about 5 times, about 5.5 times, about 6 times, about 6.5 times, about 7 times, about 8 times, or about 9 times the dose of previous basal insulin, preferably the dose of an initial or each administration of the compound of formula (I), or the pharmaceutically acceptable salt, amide, or ester thereof, is each independently about 2-20 times, preferably about 2-10 times, preferably about 2-7 times the dose of the previous basal insulin, preferably about 2 times, about 2.5 times, about 3 times, about 3.5 times, about 4 times, about 4.5 times, about 5 times, about 5.5 times, about 6 times, about 6.5 times, about 7 times, about 8 times, or about 9 times the dose of the previous basal insulin.

Further, the method comprises administering the compound of formula (I) or the pharmaceutically acceptable salt, amide, or ester thereof to the subject in need thereof with the following dose as an initial dose: about 5-400U, about 5-350U, about 5-300U, preferably about 10-250U, preferably about 10-200U, preferably about 10-150U, preferably about 10-120U, preferably about 10-110U, preferably about 10-100U, preferably about 10-90U, preferably about 15-85U, preferably about 20-80U, preferably about 20-75U, preferably about 25-75U, preferably about 30-70U, preferably about 40-70U; preferably about 10-40U; preferably about 20-40U;
preferably, the method comprises administering the compound of formula (I) or the pharmaceutically acceptable salt, amide, or ester thereof to the subject in need thereof with the following dose as the initial dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

Further, the method further comprises administering the compound of formula (I) or the pharmaceutically acceptable salt, amide, or ester thereof to the subject in need thereof at the initial dose, and then administering the compound of formula (I) or the pharmaceutically acceptable salt, amide, or ester thereof at a frequency of once every three days, twice weekly, once every four days, once every five days, once weekly or less, the dose for each administration is the same as or different from the initial dose, preferably, the dose for each administration is determined according to the individual situation of the subject.

Further, the method comprises administering the following initial dose of the compound of formula (I) or the pharmaceutically acceptable salt, amide, or ester thereof to the subject in need thereof at a frequency of once weekly: about 5-400U, preferably about 5-350U, preferably about 5-300U, preferably about 10-250U, preferably about 10-200U, preferably about 10-150U, preferably about 10-120U, preferably about 10-110U, preferably about 10-100U, preferably about 10-90U, preferably about 15-85U, preferably about 20-80U, preferably about 25-75U, preferably about 30-70U, preferably about 40-70U, preferably about 20-40U; preferably the method comprises administering to the subject in need thereof the compound of formula (I) or the pharmaceutically acceptable salt, amide, or ester thereof with the following dose as the initial dose at a frequency of once weekly: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U; preferably, for subjects who were previously administered once-daily or twice-daily basal insulin, when switching to the administration of the compound of formula (I) or the pharmaceutically acceptable salt, amide, or ester thereof, the initial dose of the administration of the compound of formula (I) or the pharmaceutically acceptable salt, amide, or ester thereof is about 2-20 times, preferably about 3-10 times, preferably about 5-7 times, the dose of previous basal insulin, preferably about 5 times, about 5.5 times, about 6 times, about 6.5 times, about 7 times, about 8 times, or about 9 times the dose of previous basal insulin;
after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-400U, preferably about 5-350U, preferably about 5-300U, preferably about 5-120U, preferably about 10-250U, preferably about 10-200U, preferably about 10-150U, preferably about 10-120U, preferably about 5-400U, preferably about 5-350U, preferably about 10-110U, preferably about 10-100U, preferably about 10-90U, preferably about 15-85U, preferably about 20-80U, preferably about 25-75U, preferably about 30-70U, preferably about 40-70U; preferably the dose M administered each week is independently about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

Further, in the method, wherein the administered dose M is adjusted weekly according to the following rules:
a) when the fasting blood glucose value of the subject is 3.9-4.3 mmol/L on the day of administration before dosing, or the lowest or average fasting capillary blood glucose value of the subject before breakfast on three consecutive occasions from two days before administration is 3.9-4.3 mmol/L, the dose M is adjusted to be reduced by about 1-30U, preferably by about 1-20U, preferably by about 3-18U, preferably by about 5-10U relative to the previous dose; or
b) when the fasting blood glucose value of the subject is 4.4-7.2 mmol/L on the day of administration before dosing, or the lowest or average fasting capillary blood glucose value of the subject before breakfast on three consecutive occasions from two days before administration is 4.4-7.2 mmol/L, the dose M is the previous dose; or
c) when the fasting blood glucose value of the subject is 7.3-8.0mmol/L on the day of administration before dosing, or the lowest or average fasting capillary blood glucose value of the subject is 7.3-8.0mmol/L before breakfast on three consecutive occasions from two days before administration, the dose M is adjusted to be increased by about 1U-30U, preferably by about 1-20U, preferably by about 3-18U, preferably by about 5-10U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 5-30U, preferably by about 15-20U, preferably by about 15-18U relative to the previous dose; or
e) when the fasting blood glucose value of the subject is ≥ 10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 10-40U, preferably by about 25-30U, preferably by about 15-18U relative to the previous dose; or
f) when the fasting blood glucose of the subject is ≤3.0mmol/L on the day of administration before dosing, or the lowest or average fasting capillary blood glucose of the subject before breakfast on three consecutive occasions from two days before administration is ≤ 3.0mmol/L, the dose is adjusted to be reduced by about 5-30U, preferably by about 15-20U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose is adjusted to be reduced by about 1-20U, preferably by about 5-10U relative to the previous dose;
h) when the fasting blood glucose of the subject is <4.4mmol/L on the day of administration before dosing, or the lowest or average fasting capillary blood glucose of the subject before breakfast on three consecutive occasions from two days before administration is <4.4mmol/L, the dose is adjusted to be reduced by about 5-20U, preferably by about 10-18U relative to the previous dose; or
i) when the fasting blood glucose value of the subject is > 7.2mmol/L on the day of administration before dosing, or the lowest or average fasting capillary blood glucose value of the subject before breakfast on three consecutive occasions from two days before administration is > 7.2mmol/L, the dose M is adjusted to be increased by about 1-30U, preferably by about 1-20U, preferably by about 3-18U relative to the previous dose.

Further, wherein the administered dose M is adjusted weekly according to the following rules:
a) when the fasting blood glucose of the subject is 3.9-4.3mmol/L on the day of administration before dosing, or the lowest or average fasting capillary blood glucose of the subject before breakfast on three consecutive occasions from two days before administration is 3.9-4.3mmol/L, the dose M is adjusted to be reduced by about 9U-18U relative to the previous dose; or
b) when the fasting blood glucose value of the subject is 4.4-7.2mmol/L on the day of administration before dosing, or the lowest or average fasting capillary blood glucose value of the subject before breakfast on three consecutive occasions from two days before administration is 4.4-7.2mmol/L, the dose M is the previous dose; or
c) when the fasting blood glucose value of the subject is 7.3-8.0mmol/L on the day of administration before dosing, or the lowest or average fasting capillary blood glucose value of the subject before breakfast on three consecutive occasions from two days before administration is 7.3-8.0mmol/L, the dose M is adjusted to be increased by about 9-18U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 18-30U relative to the previous dose; or
e) when the fasting blood glucose value of the subject is ≥ 10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 18-27U relative to the previous dose; or
f) when the fasting blood glucose value of the subject is ≤ 3.0mmol/L on the day of administration before dosing, or the lowest or average fasting capillary blood glucose value of the subject before breakfast on three consecutive occasions from two days before administration is ≤ 3.0mmol/L, the dose M is adjusted to be reduced by about 18-30U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose M is adjusted to be reduced by about 9-18U relative to the previous dose.

Further, wherein the method comprises administering the following initial dose of the compound of formula (I) or the pharmaceutically acceptable salt, amide, or ester thereof to an adult subject with type 2 diabetes who is insulin-naive and has inadequate control on oral antidiabetic drugs at a frequency of once weekly: about 10-400U, about 10-300U, about 10-100U, about 10-70U, about 20-60U, preferably about 25-55U, preferably about 28-55U, preferably about 30-50U, preferably about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U;
after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-300U, preferably about 10-250U, preferably about 10-200U, preferably about 10-150U, preferably about 5-120U, preferably about 10-120U, preferably about 10-110U, preferably about 10-100U, preferably about 30-70U, preferably about 31-67U, preferably about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 70U, about 80U, about 90U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

Further, wherein the method comprises administering the following initial dose of the compound of formula (I) or the pharmaceutically acceptable salt, amide, or ester thereof to the adult subject with type 2 diabetes who has inadequate control on a combination of oral antidiabetic drugs and basal insulin at a frequency of once weekly: about 50-90U, preferably about 50-90U, preferably about 55-85U, preferably about 60-80U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 90U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U; or the initial dose of the administration of the compound of formula (I) or the pharmaceutically acceptable salt, amide, or ester thereof is about 2-20 times, preferably about 3-10 times, preferably about 5-7 times, the dose of previous basal insulin, preferably about 2.5 times, about 3 times, about 5 times, about 5.5 times, about 6 times, about 6.5 times, about 7 times, about 8 times, or about 9 times the dose of previous basal insulin;
after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-300U, preferably about 10-250U, preferably about 20-200U, preferably about 30-150U, preferably about 40-120U, preferably about 50-110U, preferably about 50-100U, preferably about 60-100U, preferably about 61-97U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

A second aspect of the present disclosure provides a method for treating or preventing type II diabetes mellitus, wherein the method comprises administering the following initial dose of a compound of formula (I) or a pharmaceutically acceptable salt, amide, or ester thereof to an adult subject with type 2 diabetes who is insulin-naive and has inadequate control on oral antidiabetic drugs at a frequency of once weekly: about 20-60U, preferably about 25-55U, preferably about 30-50U, preferably about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, or about 50U; 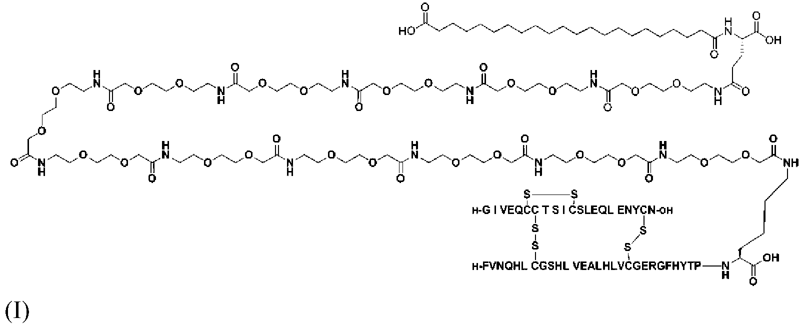
a) after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-400U, preferably about 5-350U, preferably about 5-300U, preferably about 5-120U, preferably about 10-250U, preferably about 10-200U, preferably about 10-150U, preferably about 10-120U, preferably about 5-400U, preferably about 5-350U, preferably about 10-110U, preferably about 10-100U, preferably about 10-90U, preferably about 15-85U, preferably about 20-80U, preferably about 25-75U, preferably about 30-70U, preferably about 40-70U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, the dose M is adjusted weekly according to the following rules: when the fasting blood glucose of the subject is 3.9-4.3mmol/L on the day of administration before dosing, or the lowest or average fasting capillary blood glucose of the subject before breakfast on three consecutive occasions from two days before administration is 3.9-4.3mmol/L, the dose M is adjusted to be reduced by about 9U-18U relative to the previous dose; or
b) when the fasting blood glucose value of the subject is 4.4-7.2mmol/L on the day of administration before dosing, or the lowest or average fasting capillary blood glucose value of the subject before breakfast on three consecutive occasions from two days before administration is 4.4-7.2mmol/L, the dose M is the previous dose; or
c) when the fasting blood glucose value of the subject is 7.3-8.0mmol/L on the day of administration before dosing, or the lowest or average fasting capillary blood glucose value of the subject before breakfast on three consecutive occasions from two days before administration is 7.3-8.0mmol/L, the dose M is adjusted to be increased by about 9-18U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 18-30U relative to the previous dose; or
e) when the fasting blood glucose value of the subject is ≥ 10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 18-27U relative to the previous dose; or
f) when the fasting blood glucose value of the subject is ≤ 3.0mmol/L on the day of administration before dosing, or the lowest or average fasting capillary blood glucose value of the subject before breakfast on three consecutive occasions from two days before administration is ≤ 3.0mmol/L, the dose M is adjusted to be reduced by about 18-30U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose M is adjusted to be reduced by about 9-18U relative to the previous dose.

A third aspect of the present disclosure provides a method for treating or preventing type II diabetes, wherein the method includes administering the following initial dose of a compound of formula (I) or a pharmaceutically acceptable salt, amide, or ester thereof to an adult subject with type 2 diabetes who has inadequate control on a combination of oral antidiabetic drugs and basal insulin at a frequency of once weekly: about 5-400U, about 5-350U, about 5-300U, preferably about 10-250U, preferably about 20-200U, preferably about 30-150U, preferably about 40-120U, preferably about 50-110U, preferably about 50-100U, preferably about 50-90U, preferably about 55-85U, preferably about 60-80U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U; or the initial dose of the administration of the compound of formula (I) or the pharmaceutically acceptable salt, amide, or ester thereof is about 2-20 times, preferably about 3-10 times, preferably about 5-7 times, the dose of previous basal insulin, preferably about 2.5 times, about 3 times, about 5 times, about 5.5 times, about 6 times, about 6.5 times, about 7 times, about 8 times, or about 9 times the dose of previous basal insulin; 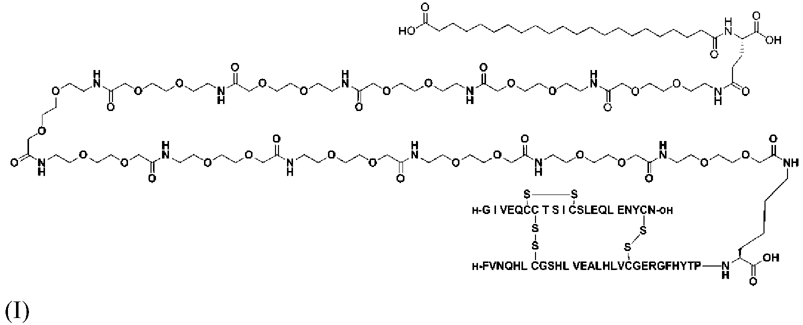 after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-400U, preferably about 5-350U, preferably about 5-300U, preferably about 5-120U, preferably about 10-250U, preferably about 10-200U, preferably about 10-150U, preferably about 10-120U, preferably about 5-400U, preferably about 5-350U, preferably about 10-110U, preferably about 10-100U, preferably about 10-90U, preferably about 15-85U, preferably about 20-80U, preferably about 25-75U, preferably about 30-70U, preferably about 40-70U, preferably about 40-70U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, the dose M is adjusted weekly according to the following rules: a) when the fasting blood glucose of the subject is 3.9-4.3mmol/L on the day of administration before dosing, or the lowest or average fasting capillary blood glucose of the subject before breakfast on three consecutive occasions from two days before administration is 3.9-4.3mmol/L, the dose M is adjusted to be reduced by about 9U-18U relative to the previous dose; or
b) when the fasting blood glucose value of the subject is 4.4-7.2mmol/L on the day of administration before dosing, or the lowest or average fasting capillary blood glucose value of the subject before breakfast on three consecutive occasions from two days before administration is 4.4-7.2mmol/L, the dose M is the previous dose; or
c) when the fasting blood glucose value of the subject is 7.3-8.0mmol/L on the day of administration before dosing, or the lowest or average fasting capillary blood glucose value of the subject before breakfast on three consecutive occasions from two days before administration is 7.3-8.0mmol/L, the dose M is adjusted to be increased by about 9-18U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 18-30U relative to the previous dose; or
e) when the fasting blood glucose value of the subject is ≥ 10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 18-27U relative to the previous dose; or
f) when the fasting blood glucose value of the subject is ≤ 3.0mmol/L on the day of administration before dosing, or the lowest or average fasting capillary blood glucose value of the subject before breakfast on three consecutive occasions from two days before administration is ≤ 3.0mmol/L, the dose M is adjusted to be reduced by about 18-30U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose M is adjusted to be reduced by about 9-18U relative to the previous dose.

Further, wherein the subject has a BMI between 18.5 kg/m² and 35 kg/m², preferably between 18.5 kg/m² and 35 kg/m²; and/or
has an HbA1c of 6.5%-10.0%, preferably 7.5%-10.0%; and/or
has a fasting venous blood glucose of ≥ 7.2mmol/L; and/or
has a fasting capillary blood glucose of ≤13.9 mmol/L; and/or
has been treated with one or more oral antidiabetic drugs; and/or
has been treated with one or more basal insulin.

Further, wherein the oral antidiabetic drugs are selected from one or more of metformin, DPP4 inhibitor, α-glycosidase inhibitor, SGLT2 inhibitor and glucokinase activator; the basal insulin is selected from one or more of insulin glargine U100, insulin detemir, insulin degludec, and human NPH insulin.

Further, in the method, wherein the same dose of oral antidiabetic drug previously used by the subject is concomitantly administered to the subject in need thereof.

Further, in the method, wherein the compound of formula (I) or the pharmaceutically acceptable salt, amide, or ester thereof is administered via parenterally, preferably subcutaneously. Further, in the method, the compound of formula (I) or the pharmaceutically acceptable salt, amide, or ester thereof is administered for 6 weeks or more, preferably 16 weeks or more, preferably 4 months or more, preferably 8 months or more, preferably 12 months or more, preferably 16 months or more, preferably 18 months or more, preferably 24 months or more, preferably 30 months or more.

Further, in the method, wherein the subject achieves a reduction in glycated hemoglobin by 0.1% or more, preferably 0.2% or more, preferably 0.3% or more, preferably 0.4% or more, preferably 0.5% or more, preferably 0.5%-30%, preferably 0.6%-25%, preferably 0.62%, 0.76%, 0.9%, 1%, 2%, 3%, 4%, 5%, 6%, 6.5%, 7%, 8%, 9%, 10%, 11%, 12%, 13%, 13.5%, 14%, 15%, 16%, 17%, 18%, 18.6%, 19%, or 20%, after administration of the compound of formula (I) or the pharmaceutically acceptable salt, amide, or ester thereof;
preferably, the subject achieves a reduction in glycated hemoglobin by 0.1% or more, after 6 weeks, 20 weeks, 25 weeks, 30 weeks, or 35 weeks of the administration of the compound of formula (I) or the pharmaceutically acceptable salt, amide, or ester thereof, preferably achieves the reduction in glycated hemoglobin of 0.2% or more, preferably 0.3% or more, preferably 0.4% or more, preferably 0.5% or more, preferably 0.5%-30%, preferably 0.6%-25%, preferably 0.62%, 0.76%, 0.9%, 1%, 2%, 3%, 4%, 5%, 6%, 6.5%, 7%, 8%, 9%, 10%, 11%, 12%, 13%, 13.5%, 14%, 15%, 16%, 17%, 18%, 18.6%, 19%, or 20%.

Further, in the method, wherein the subject achieves a reduction in fasting blood glucose by 1mM or more from baseline after administering the compound of formula (I) or the pharmaceutically acceptable salt, amide, or ester thereof, preferably by 1.2mM or more, preferably by 1.3mM, preferably by 1.4mM or more, preferably by 1.5mM or more, preferably by 1mM-10mM, preferably by 1.1mM-9.8mM, preferably by 1.2mM, 1.3mM, 1.37mM, 1.4mM, 1.5mM, 1.57mM, 1.6mM, 1.7mM, 1.77mM, 1.8mM, 1.9mM, 1.97mM, 2mM, 2.1mM, 2.2mM, 2.3mM, 2.4mM, 2.5mM, 2.6mM, 2.7mM, 2.8mM, 2.9mM, 3mM, 3.5mM, 4mM, 4.5mM, 5mM, 5.5mM, 6mM, 6.5mM, 7mM, 7.5mM, 8mM, 8.5mM, or 9mM; preferably, the subject achieves a reduction in fasting blood glucose by 1mM or more from baseline after administering the compound of formula (I) or the pharmaceutically acceptable salt, amide, or ester thereof for 6 weeks, 20 weeks, 25 weeks, 30 weeks or 35 weeks, preferably by 1.2mM or more, preferably by 1.3mM or more, preferably by 1.4mM or more, preferably by 1.5mM or more, preferably by 1mM-10mM, preferably by 1.1mM-9.8mM, preferably by 1.2mM, 1.3mM, 1.37mM, 1.4mM, 1.5mM, 1.57mM, 1.6mM, 1.7mM, 1.77mM, 1.8mM, 1.9mM, 1.97mM, 2mM, 2.1mM, 2.2mM, 2.3mM, 2.4mM, 2.5mM, 2.6mM, 2.7mM, 2.8mM, 2.9mM, 3mM, 3.5mM, 4mM, 4.5mM, 5mM, 5.5mM, 6mM, 6.5mM, 7mM, 7.5mM, 8mM, 8.5mM, or 9mM.Further, in the method, wherein the compound of formula (I), or the pharmaceutically acceptable salt, amide, or ester thereof is administered via subcutaneous injection into the abdomen, the deltoid region of the upper arm, and/or the thigh of the subject.

A fourth aspect of the present disclosure provides a kit, comprising:
a compound of formula (I), or a pharmaceutically acceptable salt, amide, or ester thereof, 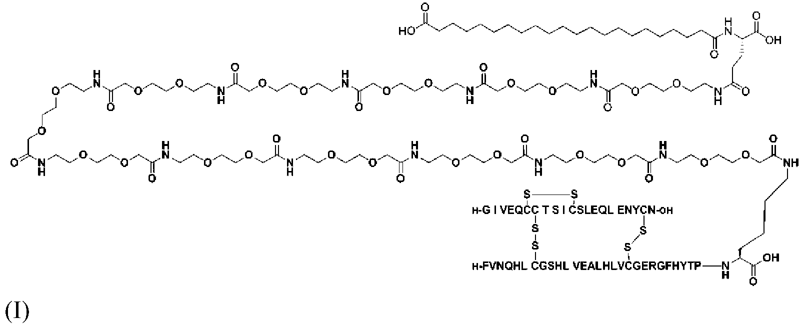
a packaging material, and
a label or package insert contained in the packaging material, which indicates that the compound of formula (I), or the pharmaceutically acceptable salt, amide, or ester thereof, is to be used for treating a subject according to any one of claims 1-22.

### BRIEF DESCRIPTION OF THE DRAWINGS

Figure 1 shows the weekly insulin doses (U/week) in group A1 and group A2 from week 1 to week 15 in Example 3.
Figure 2 shows the weekly insulin doses (U/week) in group B1 and group B2 from week 1 to week 15 in Example 4.

### Definitions

To better understand the present invention, definitions and explanations of relevant terms are provided below.

Unless otherwise specified, the terms "comprise," "include," and "have" used in this document, along with their grammatical equivalents, are generally to be understood as open-ended and non-limiting; for example, they do not exclude other elements or steps not listed herein. The term "basal insulin" refers to insulin that has a longer duration of action than endogenous or normal human insulin.

The term "insulin" comprises naturally occurring insulin, such as human insulin, as well as insulin analogs and insulin derivatives. The present invention also protects analogs and derivatives of acylated insulin that are equivalent to the compound shown in formula (I).

The term "insulin analog" comprises peptides having a molecular structure derived from a naturally occurring insulin (e.g., human insulin), which may be modified by the deletion and/or substitution (replacement) of one or more amino acid residues present in natural insulin and/or the addition of one or more amino acid residues. The added and/or substituted amino acid residues may be encodable amino acid residues, other naturally occurring amino acid residues, or purely synthetic amino acid residues. Preferably, the added and/or substituted amino acid residues are encodable amino acid residues.

The term "insulin derivative" herein refers to naturally occurring insulin or insulin analogs that have been chemically modified, such modifications may include, for example, introducing side chains at one or more positions on the insulin backbone, oxidizing or reducing groups on amino acid residues of insulin, or converting free carboxyl groups into ester groups or acylating free amino or hydroxyl groups. The acylated insulin of the present invention falls within the category of insulin derivatives.

The term "insulin parent" refers to the insulin portion of an insulin derivative or acylated insulin (also referred to herein as "parent insulin"); for example, in the present invention, it refers to the portion of an insulin derivative or acylated insulin that lacks a linked side chain or an attached acyl group. The insulin parent may be naturally occurring insulin, such as human insulin or porcine insulin. On the other hand, the insulin parent may be an insulin analog.

The term "amino acid residue" herein includes amino acids from which a hydrogen atom has been removed from the amino group and/or a hydroxyl group has been removed from the carboxyl group and/or a hydrogen atom has been removed from the thiol group. Imprecisely, amino acid residues may also be referred to as amino acids.

Unless otherwise specified, all amino acids mentioned in this application are L-amino acids.

The term "hypoglycemic event" is defined based on the lowest fasting capillary blood glucose level measured before breakfast on three consecutive occasions (including the day of the visit) starting two days prior to dosing. If the lowest fasting capillary blood glucose level measured before breakfast on three consecutive occasions (including the day of the visit) starting two days prior to dosing is ≥3.9 mmol/L, the subject is deemed not to have experienced a hypoglycemic event. Conversely, if the lowest fasting capillary blood glucose level before breakfast on three consecutive occasions (including the visit day) starting 2 days prior to dosing is <3.9 mmol/L, the subject is deemed to have experienced a hypoglycemic event.

The term "treatment" comprises therapeutic treatments, preventive treatments, and applications that reduce subjects' risk of developing diseases or other risk factors. Treatment includes, but is not limited to, the complete cure of diseases, as well as the alleviation of symptoms or mitigation of underlying risks.

The term "metabolic syndrome," as used in this application, is a recognized clinical term used to describe a condition characterized by the following combination of factors: type 2 diabetes, impaired glucose tolerance, insulin resistance, hypertension, obesity, increased waist circumference, hypertriglyceridemia, low HDL, hyperuricemia, a hypercoagulable state, and/or microalbuminuria.

The term "Type II diabetes" (also known as "Type 2 diabetes," formerly referred to as "non-insulin-dependent diabetes" or "adult-onset diabetes") accounts for 90-95% of all diabetes cases and comprises individuals with insulin resistance and a relative (rather than absolute) deficiency of insulin.

"Antidiabetic treatment" for type 2 diabetes includes:
Metformin: Metformin is typically the first prescription pharmaceutical used for type 2 diabetes. It works by increasing the body's sensitivity to insulin, allowing the body to use insulin more effectively. Metformin also reduces glucose production in the liver. Metformin alone may not lower blood sugar levels.
α-glucosidase inhibitors: α-glucosidase inhibitors delay the absorption of carbohydrates by inhibiting alpha-glucosidase in the brush border of the small intestine, thereby reducing postprandial hyperglycemia. Their main characteristics include steady blood glucose control, high safety, and the ability to reduce the incidence of cardiovascular complications; they are among the few oral hypoglycemic agents capable of intervening in impaired glucose tolerance. The most commonly used α-glucosidase inhibitors are primarily acarbose and voglibose.
Dipeptidyl peptidase-4 (DPP-4) inhibitors: These drugs also lower blood sugar levels. They do not cause weight gain. Examples of these drugs include sitagliptin, saxagliptin, vildagliptin, and linagliptin.
Sulfonylureas: These drugs help the body produce more insulin. Examples of these drugs include glibenclamide, glipizide, and glimepiride. Possible side effects include hypoglycemia and weight gain.
Thiazolidinediones: Like metformin, these drugs make the body's tissues more sensitive to insulin. These drugs are associated with weight gain and other more serious side effects, such as an increased risk of heart failure and fractures. Because of these risks, these drugs are not usually the first-line treatment. Pioglitazone is an example of a thiazolidinedione.
SGLT2 inhibitors: These are the newest antidiabetic drugs on the market. They work by preventing the kidneys from reabsorbing glucose into the bloodstream. Instead, the glucose is excreted in the urine. Examples include canagliflozin and dapagliflozin.

In this application, when referring to specific numerical values or ranges, the term "about" is generally used to mean within 20% of the given value or range, preferably within 10%, and more preferably within 5%.

The term "compound" in this application refers to a molecular entity, therefore, a "compound" may comprise various structural units in addition to the minimal unit defined for each compound or group of compounds. The term "compound" is intended to comprise its pharmaceutically relevant forms; that is, the present invention relates to the compounds defined herein or their pharmaceutically acceptable salts, amides, or esters.

The term "pharmaceutically acceptable" refers to the compounds, compositions, and/or dosage forms that, within the scope of sound medical judgment, are suitable for use in contact with human and animal tissues without causing excessive toxicity, irritation, allergic reactions, or other problems or complications, and that are commensurate with a reasonable benefit/risk ratio.

The said salt may be a basic salt, an acidic salt, or neither (i.e., a neutral salt). In water, basic salts produce hydroxide ions, while acidic salts produce hydrated hydrogen ions.

Salts of the derivatives of the present invention may be formed by adding cations or anions that react with anionic or cationic groups, respectively. These groups may be located in the peptide moiety and/or in the side chains of the derivatives of the present invention.

Non-limiting examples of anionic groups in the derivatives of the present invention include free carboxyl groups in the side chain (if any) and in the peptide moiety. The peptide moiety typically contains a free carboxyl group at the C-terminus and may also comprise free carboxyl groups at internal acidic amino acid residues such as Asp and Glu.

Non-limiting examples of cationic groups in the peptide moiety include the free amino group at the N-terminus (if any) and any free amino groups from internal basic amino acid residues such as His, Arg, and Lys.

Esters of the derivatives of the present invention may be formed, for example, by reacting a free carboxyl group with an alcohol or phenol. This reaction results in the substitution of at least one hydroxyl group with an alkoxy or aroxy group.

Ester formation may involve a free carboxyl group at the C-terminus of the peptide and/or any free carboxyl groups in the side chains.

The amides of the derivatives of the present invention may be formed, for example, by reacting a free carboxylic acid group in an activated form with an amine or a substituted amine, or by reacting a free or substituted amino group with a carboxylic acid in an activated form.

Amide formation may involve a free carboxyl group at the C-terminus of the peptide, any free carboxyl group in the side chain, the free amino group at the N-terminus of the peptide, and/or any free or substituted amino group in the peptide and/or the side chain.

In a specific embodiment, the peptide or derivative is in the form of a pharmaceutically acceptable salt. In another specific embodiment, the said derivative is in the form of a pharmaceutically acceptable amide, preferably comprising an amide group at the C-terminus of the peptide. In a further specific embodiment, the peptide or derivative is in the form of a pharmaceutically acceptable ester.

HbA1c in this application refers to glycated hemoglobin. Targets for glycated hemoglobin control are based on patient-centered, individualized principles. Currently, most diabetes guidelines recommend an HbA1c target of <7.0% for adults with type 2 diabetes mellitus (T2DM).

### Detailed Description

The following detailed description of the embodiment of the present invention will be provided with reference to specific examples. However, those skilled in the art will understand that the following examples are provided merely to illustrate the invention and should not be construed as limiting the scope of the invention. Unless otherwise specified in the examples, standard conditions or manufacturer-recommended conditions shall be followed. Unless otherwise indicated, the reagents or instruments used for which the manufacturer is not specified are all conventional commercially available products.

### Abbreviations

AE: Adverse Event
AESI: Adverse Event of Special Interest
AUC: Area Under the Concentration-time Curve
HbA1c: Glycosylated Hemoglobin
BMI: Body Mass Index
N: Number of Subjects
FPG: Fasting Plasma Glucose
SMBG: Self-Monitoring of Blood Glucose
TIR: Time in Range
GMI: Glucose Management Indicator

### Example 1: Preparation of Compound (I)

A14E, B16H, B25H, B29K (N(ε)-docosanedioyl-γGlu-12xOEG), desB30 human insulin (Compound (I), wherein the insulin parent of the compound is A14E, B16H, B25H, desB30 human insulin (SEQ ID NO: 1 and SEQ ID NO: 2, representing the A-chain and B-chain, respectively). 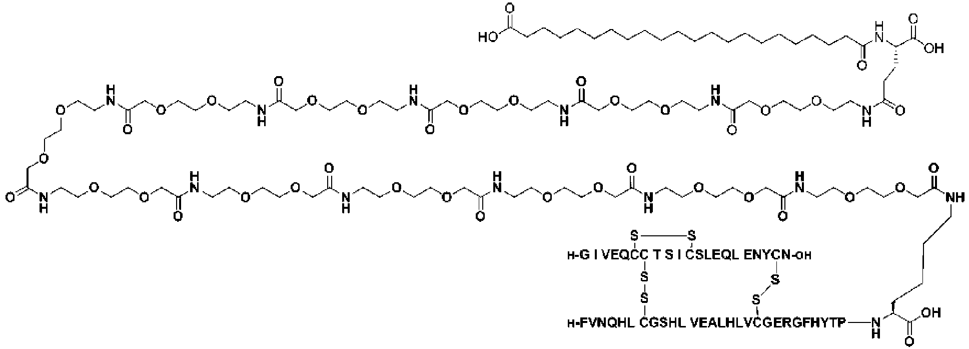
SEQ ID NO. 1:
   A14E, B16H, B25H, desB30 human insulin A-chain:
   Gly Ile Val Glu Gln Cys Cys Thr Ser Ile Cys Ser Leu Glu Gln Leu Glu Asn Tyr Cys Asn
SEQ ID NO. 2:
   A14E, B16H, B25H, desB30 human insulin B-chain:

Phe Val Asn Gln His Leu Cys Gly Ser His Leu Val Glu Ala Leu His Leu Val Cys Gly Glu Arg
Gly Phe His Tyr Thr Pro Lys

Compound (I) can be prepared, for example, according to the methods described in WO2021136296A1.

### Example 2: Injectable Formulation of Compound (I)

Formulation of a pharmaceutical composition injection of Compound (I): 1.2 mmol/L Compound (I), 4.23 mg/ml phenol, 1.08 mg/ml *m-*cresol, 1.17 mg/ml sodium chloride, 15 mg/ml glycerol, 0.71 mg/ml anhydrous disodium hydrogen phosphate, and 2.3 mol zinc ions per 6 mol of Compound (I); pH 7.4.

### Example 3: Hypoglycemic Effect of Compound (I) in Type 2 Diabetes Mellitus (Insulin-Naïve Patients with Type 2 Diabetes Inadequately Controlled on Oral Antidiabetic Drugs)

This study is a multicenter, randomized, open-label, parallel-controlled, treat-to-target Phase II clinical trial conducted in insulin-naïve patients with type 2 diabetes mellitus (T2DM) inadequately controlled on oral antidiabetic drugs. The study aims to evaluate and compare the efficacy, safety, and tolerability of once-weekly Compound (I) Injection versus once-daily insulin degludec (Tresiba^{®}) in the patient population.

### Diagnostic and Main Inclusion Criteria:

After signing the informed consent form, subjects aged 18 to 75 years (inclusive) diagnosed with T2DM for more than 6 months according to the World Health Organization (WHO) criteria (1999) and supplemented by HbA1c diagnosis were enrolled. Subjects were required to meet the following conditions to enter the trial: "7.5% ≤ HbA1c ≤ 10.0%, fasting plasma glucose ≥ 7.2 mmol/L; Body Mass Index (BMI) ≥ 18.5 kg/m² and ≤ 35 kg/m² at screening; Treatment with metformin (total daily dose ≥ 1.5 g or maximum tolerated dose ≥ 1 g) ± one other oral antidiabetic drug (DPP-4 inhibitor, alpha-glucosidase inhibitor, SGLT-2 inhibitor) with stable daily doses for ≥ 3 months prior to randomization; No prior use of insulin therapy, excluding short-term insulin treatment (maximum cumulative 14 days) prior to screening or insulin use for gestational diabetes."

Subjects meeting any of the following criteria were excluded: history of diabetic ketoacidosis, diabetic lactic acidosis, or hyperosmolar non-ketotic diabetic coma, or presence of proliferative retinopathy or maculopathy that is unstable or requires treatment within 6 months prior to screening; history of acute heart failure, or hospitalization for coronary heart disease, myocardial infarction, unstable angina, or stroke within 6 months prior to screening; history of chronic heart failure classified as New York Heart Association (NYHA) Class III or IV at screening; use of medications indicated for obesity within 3 months prior to screening or expectation to initiate or change concomitant medications known to affect body weight or glucose metabolism during the study period; history of severe hypoglycemia (Grade 3 hypoglycemia) within 6 months prior to screening, or one or more hypoglycemic events (blood glucose < 3.9 mmol/L) within 2 months prior to randomization, or recurrent symptoms related to hypoglycemia (once weekly or more) prior to randomization.

Details of the investigational drug and control drug are shown in Table 1.

**Table 1:**

| Drug | Investigational Drug | Active Control Drug |
|---|---|---|
| Formulatio n name | Compound (I) Injection | Insulin Degludec Injection (Tresiba^{®}), manufactured by Novo Nordisk (China) Pharmaceuticals Co., Ltd. |
| Specificatio n | 3 mL: 600 U (1.2 mmol/L, 3 mL/vial) | 3 mL: 300 U (FlexTouch^{®}) |
| Administrat ion Method | Subcutaneous injection (administered to an area approximately two finger-widths away from the umbilicus on the abdomen) | Subcutaneous injection (administered to an area approximately two finger-widths away from the umbilicus on the abdomen) |
| Administrat ion Method | Store at 2°C - 8°C Protect from light | Before first use: Store in a closed container at 2°C - 8°C. Protect from light and avoid freezing. |
| | | After first use or for carried spares: Do not refrigerate (store at 2°C - 8°C). Store at room temperature (not exceeding 30°C). Keep the cap on the pen to protect from light. |

As shown in Table 2, subjects were randomized and administered an initial dose (first dose) of Compound (I) Injection and Insulin Degludec Injection according to the dosing regimens specified in Table 2. All investigational drugs were administered using a 3 mL disposable pen injector. The pen injector was identical for the administration of both Compound (I) Injection and Insulin Degludec Injection.

As shown in Table 3 and Table 4, the administration of the second dose to subjects was determined based on individual patient needs. Dose adjustments were made based on the patient's fasting plasma glucose levels to optimize glycemic control, following the titration rules specified in Table 3 and Table 4.

**Table 2: Study Design: Administration of Compound (I) Injection and Insulin Degludec Injection at the Initial Stage of the Trial**

| Group | Number of Subjects (N) | Drug | Administration Method | Initial Dose | Dosing Time |
|---|---|---|---|---|---|
| Compou nd (I)-Group A | 42 | Compound (I) Injection | Once-weekly abdominal subcutaneous injection | 40 U | Once a week |
| Insulin Deglude c-Group A | 41 | Insulin Degludec Injection | Once-daily abdominal subcutaneous injection | 10 U | As per the instructions for use |

**Table 3: Weekly Insulin Dose Adjustment Rules (Absence of Hypoglycemic Events*)**

| Pre-breakfast Fasting Capillary Blood Glucose - Lowest Value or Mean from 3 Consecutive Measurements from 2 Days Prior to Dosing (mmol/L)** | Compound (I) Dose Adjustment (U/week) | Insulin Degludec Dose Adjustment (U/day) |
|---|---|---|
| 3.9-4.3 (inclusive) | -9 | - 2 |
| 4.4-7.2 (inclusive) | No adjustment | No adjustment |
| 7.3-8.0 (inclusive) | + 9 | + 2 |
| 8.1-9.9 (inclusive) | + 18 | + 4 |
| ≥10.0 | + 27 | + 6 |

| | | |
|---|---|---|
| Note: *Refers to the condition where the lowest value of pre-breakfast fasting capillary blood glucose from three consecutive measurements (including the day of the visit) over the two days prior to dosing is ≥ 3.9 mmol/L. **The lowest value of fasting capillary blood glucose should be used. If the lowest blood glucose value is ≥ 4.4 mmol/L, the mean of the three measurements should be used. If one or more fasting capillary blood glucose values are missing, dose adjustments should be based on the remaining SMBG (Subject Self-Monitoring of Blood Glucose) values. If the subject's fasting blood glucose fails to reach the predetermined treatment target, the investigator may, based on the subject's condition, individualize the insulin dose adjustment rules after communication with the sponsor. | | |

**Table 4: Weekly Insulin Dose Adjustment Rules (In the Presence of Hypoglycemic Events***)**

| Pre-breakfast Fasting Capillary Blood Glucose - Lowest Value from 3 Consecutive Measurements from 2 Days Prior to Dosing (mmol/L) | Compound (I) Dose Adjustment (U/week) | Insulin Degludec Dose Adjustment (U/day) |
|---|---|---|
| ≤3.0 | -18 | -4 |
| 3.1 (inclusive) -3.9 | - 9 | -2 |

| | | |
|---|---|---|
| Note: ***Refers to any instance where pre-breakfast fasting capillary blood glucose from three consecutive measurements (including the day of the visit) over the two days prior to dosing is < 3.9 mmol/L. | | |

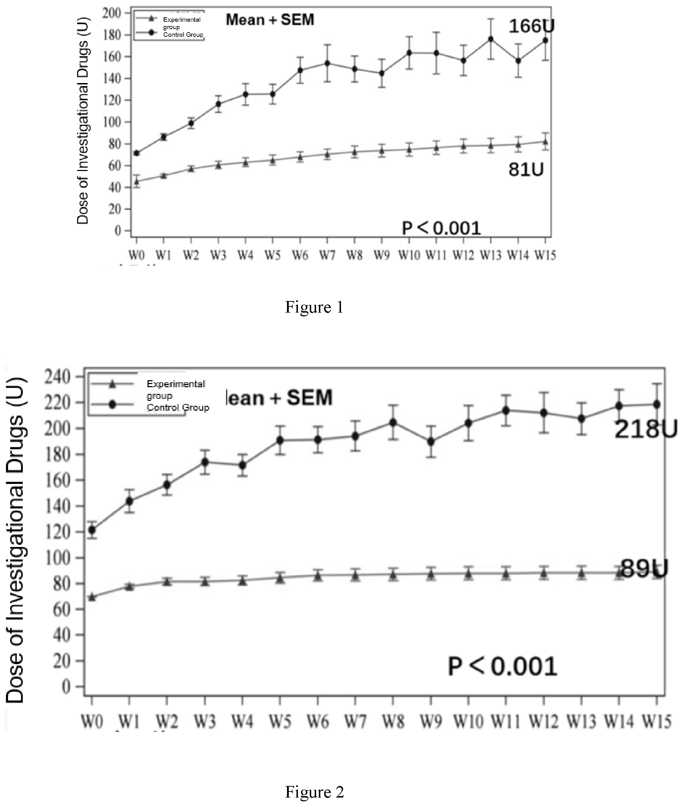

If a subject experiences fasting hypoglycemia 1-4 days after dosing, the investigator may, based on the subject's condition, individualize the insulin dose adjustment rules after communication with the sponsor. In principle, the current insulin dose should not be increased, unless there is a clear explanation for the minimum value, such as a missed meal. The specific insulin doses for the 15-week treatment are shown in Figure 1.

The total study duration for each subject was approximately 22 weeks: comprising a screening period of up to 17 days (W-2 to W-1), a 16-week treatment period (WO to W15), and a 4-week safety follow-up period (W16 to W20).

### Statistical Methods:

The primary efficacy endpoint was the change from baseline in HbA1c (%) at Week 16.

FAS (Full Analysis Set): Included all randomized subjects.

PPS (Per Protocol Set): Included all subjects without major protocol deviations who received at least 12 weeks of insulin treatment.

Primary Estimand - Main Analysis: For the FAS, an Analysis of Covariance (ANCOVA) model was used to calculate the change from baseline in HbA1c at Week 16. Treatment group and stratification factors were included as fixed effects, with age and baseline HbA1c as covariates. The change, 95% confidence interval, and p-value were calculated.

### Results:

### Subject Disposition

A total of 42 subjects were randomized to the Compound (I)-Group A, all of whom received the investigational drug, and 40 subjects (95.2%) completed the trial. A total of 41 subjects were randomized to the Insulin Degludec-Group A, all of whom received the control drug, and 38 subjects (92.7%) completed the trial.

### Baseline Characteristics

Demographic information was generally balanced between the Compound (I)-Group A and the Insulin Degludec-Group A.

### Medication Compliance Results

Compliance with the investigational drug and background medications was good. 82 subjects (98.8%) had prescribed dose compliance for the investigational drug within the 80%-120% range.

### 3.1 Efficacy

In insulin-naïve T2DM subjects inadequately controlled on oral antidiabetic drugs, once-weekly Compound (I) Injection and once-daily Insulin Degludec were administered for 16 consecutive weeks. The results showed that once-weekly Compound (I) Injection effectively reduced HbA1c, improved glycemic control, and had a generally favorable safety and tolerability profile.
(1) Overall, compared to baseline, both Group A1 and Group A2 showed a significant decrease in HbA1c, with a similar magnitude of reduction, indicating comparable glucose-lowering efficacy between the two groups.
(2) Compared to baseline, the changes in fasting plasma glucose (FPG), mean 7-point self-monitoring blood glucose (SMBG) profiles, Time in Range (TIR), and Glucose Management Indicator (GMI) were comparable between the two treatment groups.
(3) The proportion of subjects achieving the HbA1c target of ≤6.5% was significantly higher in Group A1 (38.1%) compared to Group A2 (29.3%).
(4) During the last two weeks of treatment, the mean (SD) weekly insulin doses were 80.6 U in Group A1 and 165.8 U in Group A2, with a statistically significant difference between groups (P < 0.001). The mean weekly insulin dose for Compound (I) Injection was significantly lower than that of Insulin Degludec Injection, approximately half the dose.

### HbA1c Endpoint

The primary efficacy endpoint of the study was the change from baseline in HbA1c after 16 weeks. The analysis of the primary efficacy endpoint based on the FAS is summarized in Table 5 below.

**Table 5: Analysis of Change from Baseline in HbA1c at Week 16 (FAS)**

| HbA1c (%) | Compound (I)-Group A (N=42) | Insulin Degludec-Group A (N=41) | F Value | P Value |
|---|---|---|---|---|
| Change from baseline at Week 16 | | | | |
| n (Missing data) | 40 (2) | 38 (3) | | |
| Mean (SD) | -1.355 (0.8009) | -1.297 (0.7886) | | |
| Median | -1.300 | -1.250 | | |
| Q1, Q3 | -1.850, -0.750 | -2.000, -0.800 | | |
| Min, Max | -3.00, 0.30 | -2.60, 0.30 | | |
| LSMEANS (95%CI) | -1.50 (-1.88, -1.11) | -1.48 (-1.86, -1.10) | | |
| Between-group difference LSMEANS (95% CI) | -0.02 (-0.34, 0.30) | | | |

| ANCOVA model analysis for change from baseline at Week 16 | | | | |
|---|---|---|---|---|
| Group | - | - | 0.015 | 0.902 |
| Randomization Stratification Factor | | | 0.702 | 0.499 |
| Age | | | 0.497 | 0.483 |
| Baseline HbA1c | | | 20.867 | <0.001 |

As shown in Table 5, the mean (SD) changes from baseline in HbA1c were -1.355 (0.8009)% and -1.297 (0.7886)% for the two groups, respectively. Compared to baseline, HbA1c decreased significantly in both the Compound (I)-Group A and the Insulin Degludec-Group A, with similar magnitudes of reduction, indicating comparable glucose-lowering efficacy. Once-weekly Compound (I) Injection effectively reduced HbA1c and improved glycemic control in subjects.

Based on the FAS, the primary analysis results using the ANCOVA model showed that the least squares means (LSMEANS) (95% CI) for the change from baseline in HbA1c at Week 16 were -1.50 (-1.88, -1.11)% and -1.48 (-1.86, -1.10)% for the Compound (I)-Group A and Insulin Degludec-Group A, respectively. The between-group difference (Compound (I)-Group A and Insulin Degludec-Group A) in LSMEANS (95% CI) was -0.02 (-0.34, 0.30)%, and the difference between groups was not statistically significant (P=0.902). The analysis results indicated that the effects of randomization stratification factors (stratification factors: metformin + DPP-4 inhibitor; metformin + SGLT-2 inhibitor; metformin + alpha-glucosidase inhibitor; metformin) and age were not significant (P > 0.05), while the baseline HbA1c effect was significant (P < 0.001). It can be concluded that there is no effect of the randomization stratification factors or age, but there is a baseline HbA1c effect.

### Proportion of Subjects with HbA1c ≤ 6.5% at Week 16

The study analyzed the proportion of subjects achieving the HbA1c target after 16 weeks of treatment based on the FAS. The results are shown in Table 6.

**Table 6: Proportion of Subjects with HbA1c ≤ 6.5% at Week 16 (FAS)**

| Proportion of Subjects with HbA1c ≤ 6.5% at Week 16 | Compound (I)- Group A n(%) | Insulin Degludec - Group A n(%) |
|---|---|---|
| W16-n (Missing data) | 42(0) | 41(0) |
| Number and percentage of subjects with HbA1c ≤ 6.5% at Week 16 | 16 (38.1) | 12 (29.3) |
| 95% CI for HbA1c ≤ 6.5% at Week 16 | (23.57, 54.36) | (16.13, 45.54) |
| Difference and 95% CI between Compound (I) Injection and Insulin Degludec at Week 16 | 12.93 (-5.55, 31.40) | |
| W16-EOR (95%CI) | 1.98 (0.71, 5.50) | |

As can be seen from the data in Table 6, after 16 weeks of treatment, the proportions of subjects achieving HbA1c ≤ 6.5% were 38.1% in the Compound (I)-Group A and 29.3% in the Insulin Degludec-Group A. The HbA1c ≤ 6.5% target rate in the Compound (I)-Group A was higher than that in the Insulin Degludec-Group A.

### Weekly Dose of Once-Weekly Compound (I) Injection and Once-Daily Insulin Degludec Injection During the Last 2 Weeks of the Treatment Period

The usage of the investigational drugs, Compound (I) Injection and Insulin Degludec Injection, is shown in Table 10.

**Table 10: Summary of Dosage of Investigational Drug at Visits from W14 to W15 (FAS/PPS)**

| | FAS | | PPS | |
|---|---|---|---|---|
| Investigational Drug Dose (U) | Compound (I)-Group A (N=42) | Insulin Degludec-Group A (N=41) | Compound (I)-Group A (N=40) | Insulin Degludec-Group A (N=38) |
| Actual average administered dose during W14-W15 | | | | |
| n (Missing data) | 40 (2) | 38 (3) | 40 (0) | 38 (0) |
| Mean (SD) | 80.6 (45.97) | 165.8 (100.28) | 80.6 (45.97) | 165.8 (100.28) |
| Median | 71.0 | 136.0 | 71.0 | 136.0 |
| Q1, Q3 | 53.0, 93.0 | 99.0, 210.0 | 53.0, 93.0 | 99.0, 210.0 |
| Min, Max | 24, 241 | 28, 406 | 24, 241 | 28, 406 |
| P value | <0.001 | | <0.001 | |

The results shown in Table 10 indicate that, based on the FAS, the mean (SD) of the actual average administered doses during the last two weeks of treatment (W14-W15) were 80.6 (45.97) U in the Compound (I)-Group A and 165.8 (100.28) U in the Insulin Degludec-Group A. The mean weekly insulin dose for Compound (I) was significantly lower than half that of Insulin Degludec.

The between-group comparison showed a statistically significant difference (P < 0.001). The trend observed in the PPS was consistent with that in the FAS.

Furthermore, the study found that, compared to baseline, the changes in fasting plasma glucose (FPG), mean 7-point self-monitoring blood glucose (SMBG) profiles, Time in Range (TIR), and Glucose Management Indicator (GMI) were comparable between the two treatment groups.

### 3.2 Safety

No adverse events leading to death, SUSARs, or severe hypoglycemic events occurred in the trial. According to the CTCAE (Common Terminology Criteria for Adverse Events) grading, the majority of TEAEs (Treatment-Emergent Adverse Events) were Grade 1-2 (98.14%).

No significant differences were observed in the disclosed Compound (I)-Group A regarding routine blood tests, blood biochemistry, coagulation function, urinalysis, urine biochemistry, 12-lead electrocardiogram, vital signs, physical examination, or fundus examination.

After 16 weeks of treatment in the disclosed Compound (I)-Group A, total cholesterol, low-density lipoprotein cholesterol, high-density lipoprotein cholesterol, and triglycerides showed minimal change from baseline.

### Example 4: Hypoglycemic efficacy of Compound (I) in type 2 diabetes (adult patients with type 2 diabetes inadequately controlled with oral hypoglycemic agents combined with basal insulin)

This study is a multicenter, randomized, open-label, parallel-group, treat-to-target Phase II clinical trial conducted in patients with type 2 diabetes inadequately controlled on oral hypoglycemic agents combined with basal insulin. The study aims to evaluate and compare the efficacy and safety of once-weekly Compound (I) injection versus once-daily insulin degludec in patients with type 2 diabetes inadequately controlled on oral hypoglycemic agents combined with basal insulin. The experimental materials are shown in Table 1 in Example 3.

### Diagnostic and Main Inclusion Criteria:

After subjects signed the informed consent form, this study enrolled individuals aged 18 to 75 years (inclusive) who met the World Health Organization (WHO) criteria (1999) for the diagnosis of type 2 diabetes and had a supplementary diagnosis of type 2 diabetes mellitus for more than 6 months based on glycated hemoglobin (HbA1c). Such subjects were required tomeet the following criteria at screening: 7.5% ≤ HbA1c ≤ 10.0%, fasting venous blood glucose ≥ 7.2 mmol/L; body mass index (BMI) ≥ 18.5 kg/m² and ≤ 35 kg/m²; prior to screening, they received metformin (total daily dose ≥ 1.5g or maximum tolerated dose ≥ 1g) ± one type of oral hypoglycemic agent (dipeptidyl peptidase-4 [DPP-4] inhibitor, α-glucosidase inhibitor, sodium-glucose cotransporter-2 [SGLT-2] inhibitor, glucokinase activator), and one type of basal insulin (insulin glargine U100, insulin detemir, insulin degludec, or NPH), with a basal insulin dose of 10 U to 50 U/day; the treatment regimen consisting of basal insulin and a stable dose of oral hypoglycemic agentsmust have remained unchanged for ≥3 months prior to randomization; and the basal insulin (any type) is administered once daily (QD) or twice daily (BID), among other criteria, may be enrolled in this trial.

Subjects who meet any of the following criteria were excluded: a history of diabetic ketoacidosis, diabetic lactic acidosis, or hyperosmolar nonketotic diabetic coma within 6 months prior to screening; or proliferative retinopathy or macular pathology that is unstable or requires treatment; a history of acute heart failure within 6 months prior to screening; or hospitalization within 6 months prior to screening due to coronary artery disease, myocardial infarction, unstable angina, or stroke; a history of chronic heart failure at the time of screening, classified as New York Heart Association Class III or IV; use of drugs for obesity within 3 months prior to screening, or planned initiation or modification of concomitant drugs known to affect body weight or glucose metabolism during the trial; a history of severe hypoglycemia (Grade 3 hypoglycemia) within 6 months prior to screening; or one or more episodes of hypoglycemia (blood glucose < 3.9 mmol/L) within 2 months prior to randomization; or recurrent symptoms of hypoglycemia (once or more per week) prior to randomization.

As shown in Table 11, subjects were randomized and administered the initial dose (first dose) of Compound (I) injection and insulin degludec injection according to the dosage regimen in Table 11. All drugs were administered using 3-mL disposable pen injectors. The pen injector was identical for the administration of both Compound (I) injection and insulin degludec injection.

**Table 11: Study Design: Administration of Compound (I) Injection and Insulin Degludec Injection at the Start of the study**

| **Group** | **Number of subjects (N)** | **Pharm aceutic al** | **Administration route** | **Initial dose** | **Time of administrat ion** |
|---|---|---|---|---|---|
| **Compoun d (I)-**Group B | 48 | Compo und (I) injectio n | Once-weekly subcutaneous injection in the abdomen | 70 U | At the same time every week |
| Insulin Degludec - Group B | 48 | Insulin Deglud ec Injectio n | Once-daily subcutaneous injection in the abdomen | 1 × replacement of basal insulin before randomization (according to the latest local prescribing information) | Administer as directed |

As shown in Tables 3 and 4 in Example 3, the administration of the second dose to the subject should be determined based on the individual patient's needs. Dose adjustments should be made based on the patient's fasting blood glucose level to optimize glycemic control. The adjustment rules are shown in Tables 3 and 4 in Example 3.

During the trial, the injection was administered on the same day of each week. The specific insulin doses administered over the 16-week period are shown in Figure 2.

The total study duration for each subject was up to approximately 22 weeks, comprising a screening period of up to 17 days (W-2 to W-1), a 16-week treatment period (WO~W15), and a 4-week safety follow-up period (W16~W20).

### Statistical Methods:

The primary efficacy endpoint of this study was the change from baseline in HbA1c (%) at Week 16. Assuming an estimated error for the difference in means between the two groups was 0.4%, the standard deviation for the change from baseline in HbA1c (%) at Week 16 was 0.89% for both the treatment group and the control group. Calculations were performed using PASS software (version 21.0).

With the exception of pharmacokinetic analyses, all other statistical analyses in this study were performed using SAS version 9.4 statistical software.

FAS: Included all subjects who were randomized.

PPS: Included all subjects without major protocol violations, and subjects must have received at least 12 weeks of insulin therapy.

Primary estimand-Primary Analysis: For the FAS, a covariance analysis model was used to calculate the change from baseline in HbA1c levels at Week 16, with group and stratification factors as fixed effects, age and baseline HbA1c levels as covariates, the changes, 95% confidence intervals, and p-values were calculated.

### Test Results:

### Distribution of Subjects

A total of 48 subjects were randomly enrolled into the Compound (I)-group B, all of whom received the investigational drug compound (I) injection, and 46 subjects (95.8%) completed the trial.

A total of 48 subjects were randomly enrolled into the insulin degludec-group B, all of whom received the reference investigational product insulin degludec injection, and 46 subjects (95.8%) completed the trial.

A total of 96 subjects (100.0%) were included in the Full Analysis Set (FAS) and the Safety Analysis Set (SS): 48 subjects (100.0%) in the Compound (I)-group B and 48 subjects (100.0%) in the insulin degludec-group B. A total of 87 subjects (90.6%) were included in the Per-Protocol Set (PPS): 43 subjects (89.6%) in the Compound (I)-group B and 44 subjects (91.7%) in the insulin degludec-group B.

### Baseline Characteristics

The demographic characteristics were generally comparable between the Compound (I)-group B and the insulin degludec-group B.

### Medication Adherence Results

Compliance with the investigational drug and background medication was good throughout the treatment period.

Compliance with the investigational drug was within the range of 80% to 120% for 96 subjects (100.0%).4.1 Efficacy
(1) The least squares means (LSMEANS) (95% CI) of the change from baseline in HbA1c after 16 weeks of treatment in Groups B1 and B2 were -1.26 (-1.46, -1.06) % and -0.87 (-1.07, -0.68) %, respectively. The difference in LSMEANS (95% CI) between the two groups was -0.38 (-0.66, - 0.11) %, and the comparison between groups showed a statistically significant difference (P = 0.007).
(2) After 16 weeks of treatment, the rates of achieving HbA1c < 7% and HbA1c ≤ 6.5% in Group B1 were significantly higher than those in Group B2 (Table 7).
(3) Compared with baseline, the changes in FPG, mean 7-point SMBG levels, TIR, and GMI were comparable between the two treatment groups.
(4) During the final 2 weeks of treatment, the mean (SD) weekly insulin doses for Groups B1 and B2 were 88.65 U and 218.15 U, respectively. There was a statistically significant difference between the groups (P < 0.001). The average weekly insulin dose for Compound (I) injection was significantly lower than that for insulin degludec injection, with Compound (I) injection being approximately 0.4 times that of insulin degludec injection.
(5) The median time to first achieve the titration target [the mean of three consecutive fasting capillary blood glucose readings before breakfast within the range of 4.4-7.2 mmol/L (inclusive)] was 1.14 weeks for Group B1 and 3.29 weeks for Group B2; a Kaplan-Meier analysis showed a statistically significant difference between the groups (P < 0.001).

### HbA1c endpoint

The primary efficacy endpoint of this study was the change from baseline in HbA1c levels after 16 weeks. The primary efficacy results based on the FAS analysis are shown in Table 12.

**Table 12: Analysis of Changes from Baseline in HbA1c at Week 16 (FAS)**

| **Glycated Hemoglobin (%)** | Compound (I)-**Group B** (**N=48**) | **Insulin Degludec - Group B** (**N=48**) | **F-value** | **P-value** |
|---|---|---|---|---|
| Change from baseline for W16 | | | - | - |
| n (Missing data) | 45(3) | 46(2) | | |
| Mean (SD) | -1.269(0.7856) | -0.889(0.8209) | | |
| Median | -1.200 | -0.900 | | |
| Q1, Q3 | -1.900,-0.700 | -1.400,-0.500 | | |
| Min, Max | -2.80,0.60 | -2.30,1.40 | | |
| LSMEANS(95%CI) | -1.26(-1.46,-1.06) | -0.87(-1.07,-0.68) | | |
| Difference between the two groups LSMEANS (95% CI) | -0.38(-0.66,-0.11) | | | |

| Analysis of the Covariance Model for Changes from Baseline at W16 | | | | |
|---|---|---|---|---|
| Group | | | 7.612 | 0.007 |
| Randomized stratification factors | | | 0.514 | 0.475 |
| Age | | | 15.503 | <0.001 |
| Baseline HbA1c | | | 10.755 | 0.002 |

As shown in Table 12, after 16 weeks of treatment, the mean (SD) changes from baseline in HbA1c for the two groups were -1.269 (0.7856)% and -0.889 (0.8209)%, respectively. Compared with baseline, HbA1c levels decreased significantly in both the Compound (I)-group B and the insulin degludec-group B; the magnitude of the decrease was greater in the Compound (I)-group B than in the insulin degludec-group B, indicating that Compound (I) is more effective at lowering blood glucose than insulin degludec.

Based on FAS, the main analysis results using a covariance analysis model showed that the least squares means (LSMEANS) (95% CI) for the change from baseline in HbA1c at week 16 in the Compound (I)- group B and the insulin degludec- group B were -1.26 (-1.46, -1.06) % and -0.87 (-1.07, -0.68)%, respectively. The difference in LSMEANS (95% CI) between the two groups (Compound (I)-group B and insulin degludec-group B) was -0.38 (-0.66, -0.11) %, indicating a statistically significant difference between the groups (P = 0.007). This demonstrates that the change from baseline in the compound (I)-group B was significantly greater than that in the insulin degludec-group B. The analysis results showed that the effects of the random stratification factors (stratification factors: glargine U100/ insulin degludec/insulin detemir+ HbA1c ≥ 8.5%, glargine U100/ insulin degludec/insulin detemir+ HbA1c < 8.5%, NPH + HbA1c ≥ 8.5%, NPH + HbA1c<8.5%) were not significant (P=0.475), suggesting the absence of a stratification factor effect; the effects of group, age, and baseline HbA1c were all significant (P < 0.05), indicating that group, age, and baseline HbA1c effects were present.

### Achievement rates for HbA1c < 7% and HbA1c ≤ 6.5%

The study analyzed the achievement rates of HbA1c after 16 weeks of treatment based on FAS; the results are shown in Table 13.

**Table 13 Proportion of subjects with HbA1c < 7% and ≤ 6.5% (FAS)**

| **Proportion of subjects with HbA1c <7% and** ≤**6.5% at Week 16** | **Compound (I)-Group B n(%)** | **Insulin Degludec - Group B n(%)** | **P-value** |
|---|---|---|---|
| HbA1c < 7% | | | |
| W16-n (Missing data) | 48(0) | 48(0) | |
| Number and percentage of subjects with HbA1c < 7% at Week 16 | 25(52.1) | 14(29.2) | |
| 95% CI for HbA1c < 7% at Week 16 | (37.19,66.71) | (16.95,44.06) | |

| **Proportion of subjects with HbA1c <7% and** ≤**6.5% at Week 16** | **Compound (I)-Group B n(%)** | **Insulin Degludec - Group B n(%)** | **P-value** |
|---|---|---|---|
| Difference and 95% CIbetween the compound (I) injection group and the insulin degludec injection group at Week 16 | 19.36(1.30,37.41) | | 0.038 |
| W16-EOR(95%CI) | 2.82(1.09,7.35) | | |

| HbA1c ≤6.5% | | | |
|---|---|---|---|
| W16-n (Missing data) | 48(0) | 48(0) | |
| Number and percentage of subjects with HbA1c ≤ 6.5% at Week 16 | 12(25.0) | 5(10.4) | |
| 95% CI for HbA1c ≤ 6.5% at Week 16 | (13.64,39.60) | (3.47,22.66) | |
| Difference and 95% CI between the compound (I) injection group and the insulin degludec injection group at Week 16 | 13.58(-1.18,28.34) | | 0.074 |
| W16-EOR(95%CI) | 2.83(0.88,9.07) | | |

As shown in Table 13, after 16 weeks of treatment, the rates of achieving HbA1c < 7% in the Compound (I)-group B and the insulin degludec group were 52.1% and 29.2%, respectively, while the rates of achieving HbA1c ≤ 6.5% in the Compound (I)-group B and the insulin degludec-group B were 25.0% and 10.4%, respectively. The rates of achieving HbA1c < 7% and HbA1c ≤ 6.5% in the Compound (I)-group B were significantly higher than those in the insulin degludec-group B.

### Weekly Dose of Once-Weekly Compound (I) Injection and Once-Daily Insulin Degludec Injection During the Last 2 weeks of treatment Period

The usage of the investigational drugs compound (I) injection and insulin degludec injection was studied. The results are shown in Table 17:

**Table 17 Summary of Dosages of Investigational Drugs Used in the W14-W15 Visits (FAS/PPS)**

| | FAS | | PPS | |
|---|---|---|---|---|
| Dosage of the investigational drug (U) | Compound (I)-Group B (N=48) | Insulin Degludec - Group B (N=48) | Compound (I)-Group B (N=43) | Insulin Degludec - Group(N=44) |
| Actual average dose administered during Weeks 14-15 | | | | |
| n (Missing data) | 46(2) | 46(2) | 43(0) | 44(0) |
| Mean (SD) | 88.65(34.645) | 218.15(92.603) | 87.23(34.724) | 217.84(94.707) |
| Median | 77.50 | 209.00 | 75.00 | 195.00 |
| Q1, Q3 | 66.00,108.00 | 150.00,266.00 | 64.00,106.00 | 145.00,266.25 |
| Min, Max | 20.0,172.0 | 98.0,504.0 | 20.0,172.0 | 98.0,504.0 |
| P value | <0.001 | | <0.001 | |

As shown in Table 17, during the last 2 weeks of treatment, the mean (SD) weekly insulin dose for the Compound (I)-group B and the insulin degludec-group B were 88.65 (34.645) U and 218.15 (92.603) U, respectively; there was a statistically significant difference between the two groups (P < 0.001). The average weekly insulin dose for Compound (I) was significantly lower than that for insulin degludec, approximately half the dose of insulin degludec.

Furthermore, the study found that changes in fasting plasma glucose (FPG), mean 7-point self-monitoring of blood glucose (SMBG) levels, time in range (TIR), and glucose management index (GMI) were comparable between the two treatment groups.

### 4.2 Safety

Few subjects experienced hypoglycemia at doses around 70 units. Therefore, the initial dose is approximately 70 units.

No adverse events leading to death, SUSARs, or severe hypoglycemic events occurred in this trial. According to the CTCAE (Common Terminology Criteria for Adverse Events) grading system, the majority of TEAEs were Grade 1 to 2 (97.6%).

No significant differences were observed in the blood counts, blood biochemistry, coagulation function, urinalysis, urine chemistry, 12-lead electrocardiograms, vital signs, physical examinations, or fundus examinations for the subjects in Compound (I)-group B of this study.

After 16 weeks of treatment with compound (I)-group B of the present disclosure, there were no significant changes from baseline in total cholesterol, LDL-C, HDL-C, or triglycerides.

The present invention has been described through the above examples, but it should be understood that the above examples are provided for illustrative purposes only and are not intended to limit the scope of the invention to the described examples. Furthermore, those skilled in the art will appreciate that the present invention is not limited to the above examples. Additional variations and modifications may be made based on the teachings of the present invention, and such variations and modifications fall within the scope of protection claimed herein. The scope of protection of the present invention is defined by the appended claims and their equivalents.

## Claims

1. A method for treating or preventing metabolic syndrome, wherein the method comprises administering about 5-400U, preferably about 5-120U, of a compound of formula (1), or a pharmaceutically acceptable salt, amide, or ester thereof to a subject in need thereof; preferably, the method comprises administering a dose of about 5-400U, preferably about 5-120U, of the compound of formula (I), or the pharmaceutically acceptable salt, amide, or ester thereof, to the subject in need thereof at a frequency of once every three days, twice weekly, once every four days, once every five days, once weekly or less, wherein the dose for each administration is independently the same or different, 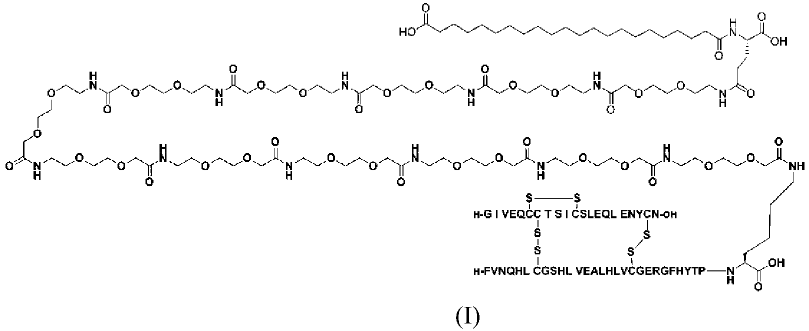
preferably, the metabolic syndrome is diabetes mellitus;
preferably, the diabetes mellitus is type II diabetes (type 2 diabetes);
preferably, the subject is a patient with type 2 diabetes who is insulin-naive and inadequately controlled on oral antidiabetic drugs;
preferably, the subject is a patient with type 2 diabetes who is inadequately controlled on a combination of oral antidiabetic drugs and basal insulin.

2. The method according to claim 1, wherein the method comprises administering to the subject in need thereof: about 5-400U, about 5-350U, about 5-300U, preferably about 10-250U, preferably about 10-200U, preferably about 10-150U, preferably about 10-120U, preferably about 10-110U, preferably about 15-105U, preferably about 20-100U, preferably about 30-100U, preferably about 31-100U, preferably about 31-97U, preferably about 35-95U, preferably about 38-90U, preferably about 40-90U, preferably about 40-88U, preferably about 45-88U, preferably about 48-85U, preferably about 49-80U, preferably about 49-79U, preferably about 49-70U, preferably about 40-70U of the compound of formula (I), or the pharmaceutically acceptable salt, amide, or ester thereof; preferably, the method comprises administering to the subject in need thereof at a frequency of once every three days, twice weekly, once every four days, once every five days, once weekly or less: about 5-400U, about 5-350U, about 5-300U, preferably about 10-250U, preferably about 10-200U, preferably about 10-150U, preferably about 10-120U, preferably about 10-110U, preferably about 15-105U, preferably about 20-100U, preferably about 30-100U, preferably about 31-100U, preferably about 31-97U, preferably about 35-95U, preferably about 38-90U, preferably about 40-90U, preferably about 40-88U, preferably about 45-88U, preferably about 48-85U, preferably about 49-80U, preferably about 49-79U, preferably about 49-70U, preferably about 40-70U of the compound of formula (I), or the pharmaceutically acceptable salt, amide, or ester thereof, wherein the dose for each administration is independently the same or different.

3. The method according to claim 1 or 2, wherein the method comprises administering to the subject in need thereof the compound of formula (I), or the pharmaceutically acceptable salt, amide, or ester thereof at a weekly dose of: about 5-300U, preferably about 10-250U, preferably about 10-200U, preferably about 10-150U, preferably about 10-120U, preferably about 10-110U, preferably about 15-105U, preferably about 20-100U, preferably about 30-100U, preferably about 31-100U, preferably about 31-97U, preferably about 35-95U, preferably about 38-90U, preferably about 40-90U, preferably about 40-88U, preferably about 45-88U, preferably about 48-85U, preferably about 49-80U, preferably about 49-79U, preferably about 49-70U, preferably about 40-70U, preferably each weekly dose is independently the same or different.

4. The method according to any one of claims 1 to 3, wherein the method comprises administering to the subject in need thereof the compound of formula (I) or the pharmaceutically acceptable salt, amide, or ester thereof at the following weekly dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, preferably each weekly dose is independently the same or different.

5. The method according to any one of claims 1 to 4, wherein the method comprises administering to the subject in need thereof the compound of formula (I) or the pharmaceutically acceptable salt, amide, or ester thereof at a frequency of once every three days, twice weekly, once every four days, once every five days, once weekly or less, preferably the dose for each administration is independently the same or different;
preferably, the method comprises administering to the subject in need thereof the following dose of the compound of formula (I) or the pharmaceutically acceptable salt, amide, or ester thereof at a frequency of once weekly: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, wherein each once-weekly dose is independently the same or different;
preferably, the method comprises administering to the subject in need thereof the compound of formula (I) or the pharmaceutically acceptable salt, amide, or ester thereof at a frequency of once weekly, at the following fixed dose based on body weight of the subject:
about 6nmol-12nmol/kg, preferably about 6nmol/kg, about 7nmol/kg, about 8nmol/kg, about 9nmol/kg, about 10nmol/kg, about 11nmol/kg, or about 12nmol/kg of the compound of formula (I) or the pharmaceutically acceptable salt, amide, or ester thereof; or
about 1U/kg-6U/kg, preferably 1U/kg-3U/kg, preferably about 1U/kg-2U/kg, preferably about 1U/kg, about 1.3U/kg, about 1.5U/kg or about 2U/kg;
preferably, for subjects who were previously administered once-daily or twice-daily basal insulin, when switching to the administration of the compound of formula (I) or the pharmaceutically acceptable salt, amide, or ester thereof, the dose of the compound of formula (I) or the pharmaceutically acceptable salt, amide, or ester thereof administered each time is about 2-20 times, preferably about 2-10 times, preferably about 2-7 times, the dose of previous basal insulin, preferably about 2 times, about 2.5 times, about 3 times, about 3.5 times, about 4 times, about 4.5 times, about 5 times, about 5.5 times, about 6 times, about 6.5 times, about 7 times, about 8 times, or about 9 times the dose of previous basal insulin, preferably the dose of an initial or each administration of the compound of formula (I), or the pharmaceutically acceptable salt, amide, or ester thereof, is each independently about 2-20 times, preferably about 2-10 times, preferably about 2-7 times the dose of the previous basal insulin, preferably about 2 times, about 2.5 times, about 3 times, about 3.5 times, about 4 times, about 4.5 times, about 5 times, about 5.5 times, about 6 times, about 6.5 times, about 7 times, about 8 times, or about 9 times the dose of the previous basal insulin.

6. The method according to any one of claims 1 to 5, wherein the method comprises administering the compound of formula (I) or the pharmaceutically acceptable salt, amide, or ester thereof to the subject in need thereof with the following dose as an initial dose: about 5-400U, about 5-350U, about 5-300U, preferably about 10-250U, preferably about 10-200U, preferably about 10-150U, preferably about 10-120U, preferably about 10-110U, preferably about 10-100U, preferably about 10-90U, preferably about 15-85U, preferably about 20-80U, preferably about 20-75U, preferably about 25-75U, preferably about 30-70U, preferably about 40-70U; preferably about 10-40U; preferably about 20-40U;
preferably, the method comprises administering the compound of formula (I) or the pharmaceutically acceptable salt, amide, or ester thereof to the subject in need thereof with the following dose as an initial dose: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

7. The method according to claim 6, wherein the method further comprises administering the compound of formula (I) or the pharmaceutically acceptable salt, amide, or ester thereof to the subject in need thereof at the initial dose, and then administering the compound of formula (I) or the pharmaceutically acceptable salt, amide, or ester thereof at a frequency of once every three days, twice weekly, once every four days, once every five days, once weekly or less, the dose for each administration is the same as or different from the initial dose, preferably, the dose for each administration is determined according to the individual situation of the subject.

8. The method according to any one of claims 1-7, wherein the method comprises administering the following initial dose of the compound of formula (I) or the pharmaceutically acceptable salt, amide, or ester thereof to the subject in need thereof at a frequency of once weekly: about 5-400U, preferably about 5-350U, preferably about 5-300U, preferably about 10-250U, preferably about 10-200U, preferably about 10-150U, preferably about 10-120U, preferably about 10-110U, preferably about 10-100U, preferably about 10-90U, preferably about 15-85U, preferably about 20-80U, preferably about 25-75U, preferably about 30-70U, preferably about 40-70U, preferably about 20-40U;
preferably the method comprises administering to the subject in need thereof the compound of formula (I) or the pharmaceutically acceptable salt, amide, or ester thereof with the following dose as the initial dose at a frequency of once weekly: about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U; preferably, for subjects who were previously administered once-daily or twice-daily basal insulin, when switching to the administration of the compound of formula (I) or the pharmaceutically acceptable salt, amide, or ester thereof, the initial dose of the administration of the compound of formula (I) or the pharmaceutically acceptable salt, amide, or ester thereof is about 2-20 times, preferably about 3-10 times, preferably about 5-7 times, the dose of previous basal insulin, preferably about 5 times, about 5.5 times, about 6 times, about 6.5 times, about 7 times, about 8 times, or about 9 times the dose of previous basal insulin;
after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-400U, preferably about 5-350U, preferably about 5-300U, preferably about 5-120U, preferably about 10-250U, preferably about 10-200U, preferably about 10-150U, preferably about 10-120U, preferably about 5-400U, preferably about 5-350U, preferably about 10-110U, preferably about 10-100U, preferably about 10-90U, preferably about 15-85U, preferably about 20-80U, preferably about 25-75U, preferably about 30-70U, preferably about 40-70U; preferably the dose M administered each week is independently about 5U, about 6U, about 7U, about 8U, about 9U, about 10U, about 11U, about 12U, about 13U, about 14U, about 15U, about 16U, about 17U, about 18U, about 19U, about 20U, about 21U, about 22U, about 23U, about 24U, about 25U, about 26U, about 27U, about 28U, about 29U, about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

9. The method according to claim 8, wherein the administered dose M is adjusted weekly according to the following rules:
a) when the fasting blood glucose value of the subject is 3.9-4.3 mmol/L on the day of administration before dosing, or the lowest or average fasting capillary blood glucose value of the subject before breakfast on three consecutive occasions from two days before administration is 3.9-4.3 mmol/L, the dose M is adjusted to be reduced by about 1-30U, preferably by about 1-20U, preferably by about 3-18U, preferably by about 5-10U relative to the previous dose; or
b) when the fasting blood glucose value of the subject is 4.4-7.2 mmol/L on the day of administration before dosing, or the lowest or average fasting capillary blood glucose value of the subject before breakfast on three consecutive occasions from two days before administration is 4.4-7.2 mmol/L, the dose M is the previous dose; or
c) when the fasting blood glucose value of the subject is 7.3-8.0mmol/L on the day of administration before dosing, or the lowest or average fasting capillary blood glucose value of the subject is 7.3-8.0mmol/L before breakfast on three consecutive occasions from two days before administration, the dose M is adjusted to be increased by about 1U-30U, preferably by about 1-20U, preferably by about 3-18U, preferably by about 5-10U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 5-30U, preferably by about 15-20U, preferably by about 15-18U relative to the previous dose; or
e) when the fasting blood glucose value of the subject is ≥10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 10-40U, preferably by about 25-30U, preferably by about 15-18U relative to the previous dose; or
f) when the fasting blood glucose of the subject is ≤3.0mmol/L on the day of administration before dosing, or the lowest or average fasting capillary blood glucose of the subject before breakfast on three consecutive occasions from two days before administration is ≤ 3.0mmol/L, the dose is adjusted to be reduced by about 5-30U, preferably by about 15-20U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose is adjusted to be reduced by about 1-20U, preferably by about 5-10U relative to the previous dose;
h) when the fasting blood glucose of the subject is <4.4mmol/L on the day of administration before dosing, or the lowest or average fasting capillary blood glucose of the subject before breakfast on three consecutive occasions from two days before administration is <4.4mmol/L, the dose is adjusted to be reduced by about 5-20U, preferably by about 10-18U relative to the previous dose; or
i) when the fasting blood glucose value of the subject is > 7.2mmol/L on the day of administration before dosing, or the lowest or average fasting capillary blood glucose value of the subject before breakfast on three consecutive occasions from two days before administration is > 7.2mmol/L, the dose M is adjusted to be increased by about 1-30U, preferably by about 1-20U, preferably by about 3-18U relative to the previous dose.

10. The method according to claim 8 or 9, wherein the administered dose M is adjusted weekly according to the following rules:
a) when the fasting blood glucose of the subject is 3.9-4.3mmol/L on the day of administration before dosing, or the lowest or average fasting capillary blood glucose of the subject before breakfast on three consecutive occasions from two days before administration is 3.9-4.3mmol/L, the dose M is adjusted to be reduced by about 9U-18U relative to the previous dose; or
b) when the fasting blood glucose value of the subject is 4.4-7.2mmol/L on the day of administration before dosing, or the lowest or average fasting capillary blood glucose value of the subject before breakfast on three consecutive occasions from two days before administration is 4.4-7.2mmol/L, the dose M is the previous dose; or
c) when the fasting blood glucose value of the subject is 7.3-8.0mmol/L on the day of administration before dosing, or the lowest or average fasting capillary blood glucose value of the subject before breakfast on three consecutive occasions from two days before administration is 7.3-8.0mmol/L, the dose M is adjusted to be increased by about 9-18U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 18-30U relative to the previous dose; or
e) when the fasting blood glucose value of the subject is ≥10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 18-27U relative to the previous dose; or
f) when the fasting blood glucose value of the subject is ≤ 3.0mmol/L on the day of administration before dosing, or the lowest or average fasting capillary blood glucose value of the subject before breakfast on three consecutive occasions from two days before administration is ≤ 3.0mmol/L, the dose M is adjusted to be reduced by about 18-30U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose M is adjusted to be reduced by about 9-18U relative to the previous dose.

11. The method according to any one of claims 8 to 10, wherein the method comprises administering the following initial dose of the compound of formula (I) or the pharmaceutically acceptable salt, amide, or ester thereof to an adult subject with type 2 diabetes who is insulin-naive and has inadequate control on oral antidiabetic drugs at a frequency of once weekly: about 10-400U, about 10-300U, about 10-100U, about 10-70U, about 20-60U, preferably about 25-55U, preferably about 28-55U, preferably about 30-50U, preferably about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U;
after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-300U, preferably about 10-250U, preferably about 10-200U, preferably about 10-150U, preferably about 5-120U, preferably about 10-120U, preferably about 10-110U, preferably about 10-100U, preferably about 30-70U, preferably about 31-67U, preferably about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, about 50U, about 51U, about 52U, about 53U, about 54U, about 55U, about 56U, about 57U, about 58U, about 59U, about 60U, about 61U, about 62U, about 63U, about 64U, about 65U, about 66U, about 67U, about 70U, about 80U, about 90U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

12. The method according to any one of claims 8 to 11, wherein the method comprises administering the following initial dose of the compound of formula (I) or the pharmaceutically acceptable salt, amide, or ester thereof to the adult subject with type 2 diabetes who has inadequate control on a combination of oral antidiabetic drugs and basal insulin at a frequency of once weekly: about 50-90U, preferably about 50-90U, preferably about 55-85U, preferably about 60-80U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 90U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U; or the initial dose of the administration of the compound of formula (I) or the pharmaceutically acceptable salt, amide, or ester thereof is about 2-20 times, preferably about 3-10 times, preferably about 5-7 times, the dose of previous basal insulin, preferably about 2.5 times, about 3 times, about 5 times, about 5.5 times, about 6 times, about 6.5 times, about 7 times, about 8 times, or about 9 times the dose of previous basal insulin;
after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-300U, preferably about 10-250U, preferably about 20-200U, preferably about 30-150U, preferably about 40-120U, preferably about 50-110U, preferably about 50-100U, preferably about 60-100U, preferably about 61-97U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U.

13. A method for treating or preventing type II diabetes mellitus, wherein the method comprises administering the following initial dose of a compound of formula (I) or a pharmaceutically acceptable salt, amide, or ester thereof to an adult subject with type 2 diabetes who is insulin-naive and has inadequate control on oral antidiabetic drugs at a frequency of once weekly: about 20-60U, preferably about 25-55U, preferably about 30-50U, preferably about 30U, about 31U, about 32U, about 33U, about 34U, about 35U, about 36U, about 37U, about 38U, about 39U, about 40U, about 41U, about 42U, about 43U, about 44U, about 45U, about 46U, about 47U, about 48U, about 49U, or about 50U; 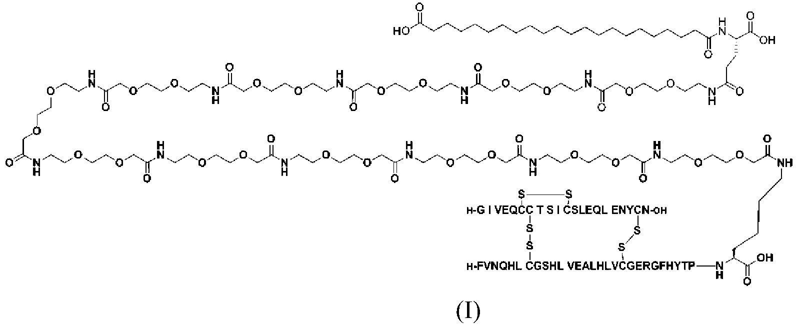 after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-400U, preferably about 5-350U, preferably about 5-300U, preferably about 5-120U, preferably about 10-250U, preferably about 10-200U, preferably about 10-150U, preferably about 10-120U, preferably about 5-400U, preferably about 5-350U, preferably about 10-110U, preferably about 10-100U, preferably about 10-90U, preferably about 15-85U, preferably about 20-80U, preferably about 25-75U, preferably about 30-70U, preferably about 40-70U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, the dose M is adjusted weekly according to the following rules:
a) when the fasting blood glucose of the subject is 3.9-4.3mmol/L on the day of administration before dosing, or the lowest or average fasting capillary blood glucose of the subject before breakfast on three consecutive occasions from two days before administration is 3.9-4.3mmol/L, the dose M is adjusted to be reduced by about 9U-18U relative to the previous dose; or
b) when the fasting blood glucose value of the subject is 4.4-7.2mmol/L on the day of administration before dosing, or the lowest or average fasting capillary blood glucose value of the subject before breakfast on three consecutive occasions from two days before administration is 4.4-7.2mmol/L, the dose M is the previous dose; or
c) when the fasting blood glucose value of the subject is 7.3-8.0mmol/L on the day of administration before dosing, or the lowest or average fasting capillary blood glucose value of the subject before breakfast on three consecutive occasions from two days before administration is 7.3-8.0mmol/L, the dose M is adjusted to be increased by about 9-18U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 18-30U relative to the previous dose; or
e) when the fasting blood glucose value of the subject is ≥ 10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 18-27U relative to the previous dose; or
f) when the fasting blood glucose value of the subject is ≤ 3.0mmol/L on the day of administration before dosing, or the lowest or average fasting capillary blood glucose value of the subject before breakfast on three consecutive occasions from two days before administration is ≤ 3.0mmol/L, the dose M is adjusted to be reduced by about 18-30U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose M is adjusted to be reduced by about 9-18U relative to the previous dose.

14. A method for treating or preventing type II diabetes, the method includes administering the following initial dose of a compound of formula (I) or a pharmaceutically acceptable salt, amide, or ester thereof to an adult subject with type 2 diabetes who has inadequate control on a combination of oral antidiabetic drugs and basal insulin at a frequency of once weekly: about 5-400U, about 5-350U, about 5-300U, preferably about 10-250U, preferably about 20-200U, preferably about 30-150U, preferably about 40-120U, preferably about 50-110U, preferably about 50-100U, preferably about 50-90U, preferably about 55-85U, preferably about 60-80U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U; or the initial dose of the administration of the compound of formula (I) or the pharmaceutically acceptable salt, amide, or ester thereof is about 2-20 times, preferably about 3-10 times, preferably about 5-7 times, the dose of previous basal insulin, preferably about 2.5 times, about 3 times, about 5 times, about 5.5 times, about 6 times, about 6.5 times, about 7 times, about 8 times, or about 9 times the dose of previous basal insulin; 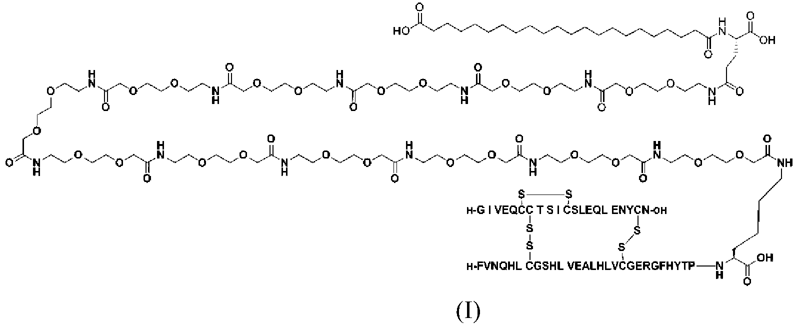 after the initial dose is administered for 1 week, the compound of formula (I) or the pharmaceutically acceptable salt, amide, or ester thereof is administered at a dose of M according to the subject's blood glucose condition at a frequency of once weekly, the dose M administered each week is the same or different, which is independently about 5-400U, preferably about 5-350U, preferably about 5-300U, preferably about 5-120U, preferably about 10-250U, preferably about 10-200U, preferably about 10-150U, preferably about 10-120U, preferably about 5-400U, preferably about 5-350U, preferably about 10-110U, preferably about 10-100U, preferably about 10-90U, preferably about 15-85U, preferably about 20-80U, preferably about 25-75U, preferably about 30-70U, preferably about 40-70U, preferably about 40-70U, preferably about 60U, about 61U, about 62U, about 63U, about 64U, about 65U, about 66U, about 67U, about 68U, about 69U, about 70U, about 71U, about 72U, about 73U, about 74U, about 75U, about 76U, about 77U, about 78U, about 79U, about 80U, about 81U, about 82U, about 83U, about 84U, about 85U, about 86U, about 87U, about 88U, about 89U, about 90U, about 91U, about 92U, about 93U, about 94U, about 95U, about 96U, about 97U, about 98U, about 99U, about 100U, about 110U, about 120U, about 130U, about 140U, about 150U, about 160U, about 170U, about 180U, about 190U, about 200U, about 210U, about 220U, about 230U, about 240U, about 250U, about 260U, about 270U, about 280U, about 290U, or about 300U, the dose M is adjusted weekly according to the following rules:
a) when the fasting blood glucose of the subject is 3.9-4.3mmol/L on the day of administration before dosing, or the lowest or average fasting capillary blood glucose of the subject before breakfast on three consecutive occasions from two days before administration is 3.9-4.3mmol/L, the dose M is adjusted to be reduced by about 9U-18U relative to the previous dose; or
b) when the fasting blood glucose value of the subject is 4.4-7.2mmol/L on the day of administration before dosing, or the lowest or average fasting capillary blood glucose value of the subject before breakfast on three consecutive occasions from two days before administration is 4.4-7.2mmol/L, the dose M is the previous dose; or
c) when the fasting blood glucose value of the subject is 7.3-8.0mmol/L on the day of administration before dosing, or the lowest or average fasting capillary blood glucose value of the subject before breakfast on three consecutive occasions from two days before administration is 7.3-8.0mmol/L, the dose M is adjusted to be increased by about 9-18U relative to the previous dose; or
d) when the fasting blood glucose value of the subject is 8.1-9.9mmol/L on the day of administration before dosing, or the lowest or average fasting capillary blood glucose value of the subject before breakfast on three consecutive occasions from two days before administration is 8.1-9.9mmol/L, the dose M is adjusted to be increased by about 18-30U relative to the previous dose; or
e) when the fasting blood glucose value of the subject is ≥10.0mmol/L on the day of administration before dosing, or the lowest or average fasting capillary blood glucose value of the subject before breakfast on three consecutive occasions from two days before administration is ≥ 10.0mmol/L, the dose M is adjusted to be increased by about 18-27U relative to the previous dose; or
f) when the fasting blood glucose value of the subject is ≤ 3.0mmol/L on the day of administration before dosing, or the lowest or average fasting capillary blood glucose value of the subject before breakfast on three consecutive occasions from two days before administration is ≤ 3.0mmol/L, the dose M is adjusted to be reduced by about 18-30U relative to the previous dose; or
g) when the fasting blood glucose value of the subject is 3.1-3.9mmol/L on the day of administration before dosing, or the lowest or average fasting capillary blood glucose value of the subject before breakfast on three consecutive occasions from two days before administration is 3.1-3.9mmol/L, the dose M is adjusted to be reduced by about 9-18U relative to the previous dose.

15. The method according to any one of claims 1 to 14, wherein the subject has a BMI between 18.5 kg/m² and 35 kg/m², preferably between 18.5 kg/m² and 35 kg/m²; and/or
has an HbA1c of 6.5%-10.0%, preferably 7.5%-10.0%; and/or
has a fasting venous blood glucose of ≥ 7.2mmol/L; and/or
has a fasting capillary blood glucose of ≤13.9 mmol/L; and/or
has been treated with one or more oral antidiabetic drugs; and/or
has been treated with one or more basal insulin.

16. The method according to claim 15, wherein the oral antidiabetic drugs are selected from one or more of metformin, DPP4 inhibitor, α-glycosidase inhibitor, SGLT2 inhibitor and glucokinase activator; the basal insulin is selected from one or more of insulin glargine U100, insulin detemir, insulin degludec, and human NPH insulin.

17. The method according to any one of claims 1 to 16, wherein the same dose of oral antidiabetic drug previously used by the subject is concomitantly administered to the subject in need thereof.

18. The method according to any one of claims 1 to 17, wherein the compound of formula (I) or the pharmaceutically acceptable salt, amide, or ester thereof is administered via parenterally, preferably subcutaneously.

19. The method according to any one of claims 1 to 18, wherein the compound of formula (I) or the pharmaceutically acceptable salt, amide, or ester thereof is administered for 6 weeks or more, preferably 16 weeks or more, preferably 4 months or more, preferably 8 months or more, preferably 12 months or more, preferably 16 months or more, preferably 18 months or more, preferably 24 months or more, preferably 30 months or more.

20. The method according to any one of claims 1 to 19, wherein the subject achieves a reduction in glycated hemoglobin by 0.1% or more, preferably 0.2% or more, preferably 0.3% or more, preferably 0.4% or more, preferably 0.5% or more, preferably 0.5%-30%, preferably 0.6%-25%, preferably 0.62%, 0.76%, 0.9%, 1%, 2%, 3%, 4%, 5%, 6%, 6.5%, 7%, 8%, 9%, 10%, 11%, 12%, 13%, 13.5%, 14%, 15%, 16%, 17%, 18%, 18.6%, 19%, or 20%, after administration of the compound of formula (I) or the pharmaceutically acceptable salt, amide, or ester thereof;
preferably, the subject achieves a reduction in glycated hemoglobin by 0.1% or more, after 6 weeks, 20 weeks, 25 weeks, 30 weeks, or 35 weeks of the administration of the compound of formula (I) or the pharmaceutically acceptable salt, amide, or ester thereof, preferably achieves the reduction in glycated hemoglobin of 0.2% or more, preferably 0.3% or more, preferably 0.4% or more, preferably 0.5% or more, preferably 0.5%-30%, preferably 0.6%-25%, preferably 0.62%, 0.76%, 0.9%, 1%, 2%, 3%, 4%, 5%, 6%, 6.5%, 7%, 8%, 9%, 10%, 11%, 12%, 13%, 13.5%, 14%, 15%, 16%, 17%, 18%, 18.6%, 19%, or 20%.

21. The method according to any one of claims 1 to 19, wherein the subject achieves a reduction in fasting blood glucose by 1mM or more from baseline after administering the compound of formula (I) or the pharmaceutically acceptable salt, amide, or ester thereof, preferably by 1.2mM or more, preferably by 1.3mM, preferably by 1.4mM or more, preferably by 1.5mM or more, preferably by 1mM-10mM, preferably by 1.1mM-9.8mM, preferably by 1.2mM, 1.3mM, 1.37mM, 1.4mM, 1.5mM, 1.57mM, 1.6mM, 1.7mM, 1.77mM, 1.8mM, 1.9mM, 1.97mM, 2mM, 2.1mM, 2.2mM, 2.3mM, 2.4mM, 2.5mM, 2.6mM, 2.7mM, 2.8mM, 2.9mM, 3mM, 3.5mM, 4mM, 4.5mM, 5mM, 5.5mM, 6mM, 6.5mM, 7mM, 7.5mM, 8mM, 8.5mM, or 9mM; preferably, the subject achieves a reduction in fasting blood glucose by 1mM or more from baseline after administering the compound of formula (I) or the pharmaceutically acceptable salt, amide, or ester thereof for 6 weeks, 20 weeks, 25 weeks, 30 weeks or 35 weeks, preferably by 1.2mM or more, preferably by 1.3mM or more, preferably by 1.4mM or more, preferably by 1.5mM or more, preferably by 1mM-10mM, preferably by 1.1mM-9.8mM, preferably by 1.2mM, 1.3mM, 1.37mM, 1.4mM, 1.5mM, 1.57mM, 1.6mM, 1.7mM, 1.77mM, 1.8mM, 1.9mM, 1.97mM, 2mM, 2.1mM, 2.2mM, 2.3mM, 2.4mM, 2.5mM, 2.6mM, 2.7mM, 2.8mM, 2.9mM, 3mM, 3.5mM, 4mM, 4.5mM, 5mM, 5.5mM, 6mM, 6.5mM, 7mM, 7.5mM, 8mM, 8.5mM, or 9mM.

22. The method according to any one of claims 1 to 21, wherein the compound of formula (I), or the pharmaceutically acceptable salt, amide, or ester thereof is administered via subcutaneous injection into the abdomen, the deltoid region of the upper arm, and/or the thigh of the subject.

23. A kit, comprising:
a compound of formula (I), or a pharmaceutically acceptable salt, amide, or ester thereof, 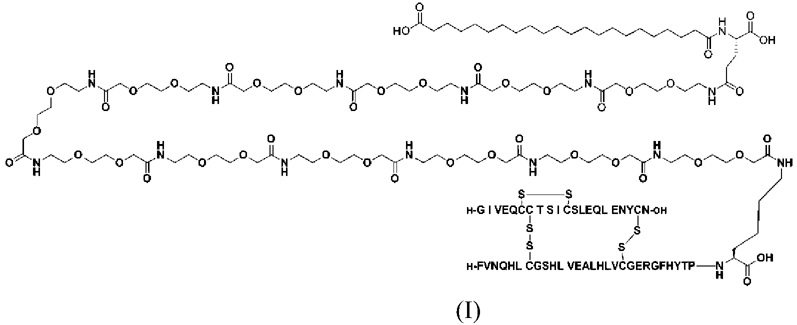
a packaging material, and
a label or package insert contained in the packaging material, which indicates that the compound of formula (I), or the pharmaceutically acceptable salt, amide, or ester thereof, is to be used for treating a subject according to any one of claims 1 to 22.
